# EUROPEAN PATENT APPLICATION

(11) **EP 1 785 427 A1**
(43) Date of publication of application: **16.05.2007**
(21) Application number: 05077580.8
(22) Date of filing: 10.11.2005
(51) Int. Cl.: C07D 491/22

(54) **Syntesis of tetrodotoxin, its analogues and intermediates thereof**

(71) Applicant: LABORATORIOS DEL DR. ESTEVE, S.A., 08041 Barcelona (ES)
(72) Inventor: Noheda Marin, Pedro, Juan de la Cierva 28006,Madrid (ES)
(74) Representative: ABG Patentes, S.L.

(57) **Abstract**

The invention provides for the synthesis of Tetrodotoxin, its tautomers, enantiomers, distercoisomer or analogues through the use of spirolactam compounds of formula II 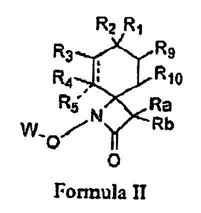 as starting material, by incorporation of a guanidine (or similar) residue to a compound of formula II prior to the formation of an orthoester, or the use of a compound of formula VII as starting material.

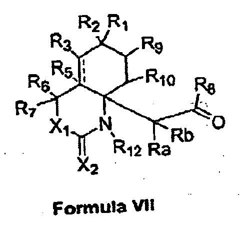

## Description

### FIELD OF THE INVENTION

The present invention relates to a synthesis of Tetrodotoxin and analogues thereof. New compounds useful as intermediates are also disclosed.

### BACKGROUND OF THE INVENTION

In the present application the following numbering will be used (see figure 1, page 7497 in Sato, K.; Akai, S.; Sugita, N.; Ohsawa, T.; Kogure, T.; Shoji, H.; Yoshimura, J. J. Org. Chem. 2005, 70, 7496-7504):

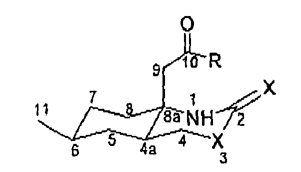

Tetrodotoxin (TTX), Octahydro-12-(hydroxymethyl)-2-imino-5,9:7, 10a-dimethano-10aH-[1,3]dioxocino[6,5-d]pyrimidine-4,7,10,11,12-pentol, is a potent neurotoxin which selectively binds to sodium channel proteins inhibiting its function in the cell membrane. Tetrodotoxin was first isolated in 1909 from the ovaries of the puffer fish, and named after the puffer fish family "Tetraodontidae". Despite being a small molecule, it has an extremely complex structure characterised by a dioxaadamantane skeleton, a guanidine residue at C2 which is part of a hemiaminal at C4, and an orthoacid bridge at C10.

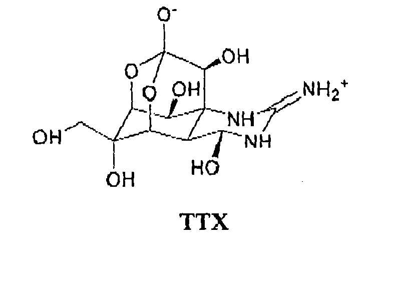

Due to its novel structure and high density of functional groups, only after extensive efforts, Hirata-Goto *et al.* (Goto, T.; Kishi, Y.; Takahashi, S.; Hirata, Y. Tetrahedron 1965, 21, 2059-2088), Tsuda *et al,* (Tsuda, K.; Tkuma, S.; Kawamura, M.; Tachikawa, K.; Sakai, K.; Tamura, C.; Amakasu, O. Chem. Pharm. Bull. 1964, 12, 1357-1374) and Woodward *et al.* (Woodward, R. B. Pure Appl. Chem. 1964, 9, 49-74; Woodward, R. B.; Gougoutas, J. Z. J. Am. Chem. Soc. 1964, 86, 5030) independently arrived at the same structure in 1964. Finally, in 1970 absolute stereochemistry was determined unambiguously by X-ray crystallographic analysis of its derivative (Furusaki, A.; Tomic, Y.; Nitta, 1. Bull. Chem. Soc. Jpn. 1970, 43, 3325-3331).

Tetrodotoxin blocks diffusion of sodium through the sodium channel, preventing depolarization and propagation of action potentials in nerve cells. The TTX-Na Channel binding site is extremely tight (*K_{d}* = 10⁻¹⁰ nM). Therefore it is an essential tool in pharmacological studies related to sodium channel proteins.

Further, Tetrodotoxin, due to its analgesic properties, is a promising new drug candidate in the field of pain management. It is currently in phase III clinical trials for cancer pain.

Extraction and purification of natural TTX is complicated, and dependent on the availability of the right animal source. Therefore there is an existing need of providing larger amounts of Tetrodotoxin and its analogues, and researchers seek new cost effective and efficient synthesis. Up to the date, only a handfull of total synthesis has been reported.

The first total synthesis of Tetrodotoxin was reported by Kishi in 1972 and afforded Tetrodotoxin as a racemic mixture after 29 steps in a 0.66% overall yield (Kishi, Y.; Nakatsubo, F.; Aratani, M.; Goto, T.; Inoue, S.; Kakoi, H.; Tetrahedron Lett. 1970, 59, 5127-5128; Kishi, Y.; Nakatsubo, F.; Aratani, M.; Goto, T.; Inoue, S.; Kakoi, 11.; Tanino, H.; Sugiura, S. J. Am. Chem. Soc. 1972, 94, 9217-9219; Kishi, Y.; Nakatsubo, F.; Aratani, M.; Goto, T.; Inoue, S.; Kakoi, H.; Tanino, H.; Sugiura, S. J. Am. Chem. Soc. 1972, 94, 9219-9220; Kishi, Y.; Nakatsubo, F.; Aratani, M.; Goto, T.; Inoue, S.; Kakoi, H.; Tanino, H.; Sugiura, S. J. Am. Chem. Soc. 1972, 94, 9220-9221).

The first asymmetric synthesis of Tetrodotoxin was reported by Isobe *et al*. in January 2003 (Isobe,M. et al, J. Am. Chem. Soc, 2003, 125, 8798-8805). This synthesis relied in a Claissen rearrangement, a Shonogashira coupling and an intramolecular carbamate-ester conjugate addition as key steps. After 67 steps Tetrodotoxin was obtained in a 1,22% overall yield.

Shortly after (June, 2003) Dubois *et al.* reported a second asymmetric synthesis of Tetrodotoxin having a Rhodium-catalazed carbene and nitrene insertion as key step with an overall yield of 0.49% after 32 steps (Du Bois, J-; Hinman, A. J. Am. Chem. Soc. 2003, 125, 11510-11511).

Recently, Isobe *et al,* have reported an additional asymmetric total synthesis comprising 62 steps and an overall yield of approximately 1% from a vinilic methyl intermediate (Isobe, M.; Urabe, D.; Nishikawa, T. Angew. Chem. Int. Ed. 2004, 43, 4782-4785).

Also, Isobe has reported the synthesis of different unnatural analogues of Tetrodotoxin following similar strategies as those reported for Tetrodotoxin. Concretely, Isobe *et al,* has reported the synthesis of (-)-5,11-Dideoxytetrodotoxin (Isobe, M.; Asai, M.; Ohyabu, N.; Yamamoto, N.; Nishikawa, T. Angew. Chem. Int. Ed. 1999, 38, 3081-3084), (-)-8,11-Dideoxytetrodotoxin (Isobe, M.; Asai, M.; Iwabuchi, T.; Yoshida, K.; Urabe, D., Nishikawa, T. Org. Lett. 2002, 16, 2679-2682; Isobe, M.; Asai, M.; Iwabuchi, T.; Yoshida, K.; Urabe, D.; Nishikawa, T. Chem. Eur. J. 2004, 10, 452-462) and 11-Deoxytetrodotoxin (Isobe, M.; Asai, Nishikawa, T. J. Am. Chem- Soc. 2002, 124, 7847-7852).

Finally, Sato *et al.* have recently reported an asymmetric total synthesis of Tetrodotoxin from *myo*-inositol in approximately 0.14% overall yield after 40 steps (Sato, K.; Akai, S.; Sugita, N.; Ohsawa, T.; Kogure, T.; Shoji, H.; Yoshimura, J. J. Org. Chem. 2005, 70, 7496-7504).

Also, further discussions regarding different synthetic approaches to Tetrodotoxin and its analogues may be reviewed in Koert, U. T. Angew. Chem. Int. Ed. 2004, 43, 5572-55769.

In view of all of the above there is an existing need of providing an alternative cost effective and efficient synthesis of Tetradotoxin and analogues thereof, which can be used for the industrial production of sufficient amounts of these compounds. Further, it will open the way to new analogues with useful biological and pharmacological properties.

As a general trend, all the above mentioned synthesis start with the construction of a highly functionalized cyclohexane, which is used as the template over which, first, the ortocsther (or an immediate precursor such as a lactone) and, then, the six-membered ring having the guanidine residue, are built (Figure I). Due to the high polarity of the orthoester and the guanidine group, their introduction in the molecule is usually delayed until the last steps of the synthesis.

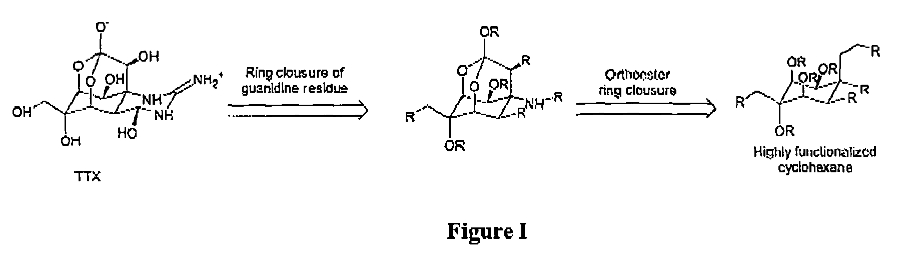

### SUMMARY OF THE INVENTION

It has know been surprisingly found that the compounds described in our applications EP 04076477.1 and PCT/EP2005/005149, which are herein incorporated by reference in their entirety, are suitable precursors for the synthesis of Tetrodotoxin and analogues thereof. These compounds, having the following formula: 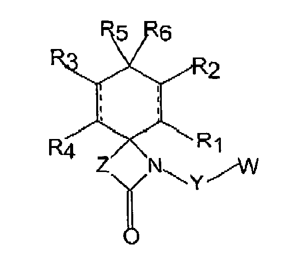 wherein R₁, R₂, R₃ and R₄ are each independently selected from H, OH, halo, OPr, -O, cyano, substituted or unsubstituted alkyl, substituted or unsubstituted aryl, substituted or unsubstituted heterocyclyl, substituted or unsubstituted alkoxy, substituted or unsubstituted aryloxy, substituted or unsubstituted amino or halogen,
R₅ and R₆ together are =O or -O-(CH₂)ₘ-, wherein m is selected from 1, 2, 3, 4 or 5;
or R₅ is selected from H, OH, OPr and R₆, is selected from hydrogen, cyano, substituted or unsubstituted alkyl, substituted or unsubstituted cycloalkyl, substituted or unsubstituted alkenyl, substituted or unsubstituted alkinyl, substituted or unsubstituted aryl, substituted or unsubstituted heterocyclyl,
with the proviso that at least one of R₁, R₂, R₃, R₄ or R₅ is OH, substituted or unsubstituted alkoxy, substituted or unsubstituted aryloxy or OPr;
Pr is an hydroxyl protecting group that can be the same or different on each of R₁, R₂, R₃, R₄ or R₅ and that can simultaneously protect 1, 2 or 3 hydroxy groups;
the dotted line represents a single or double bond, with the proviso that when both R₁ and R₂ or R₃ and R₄ are H then there is a double bond between the two C to which the H are linked;
Z is -(CRaRb)ₙ- or -CH₂-(CRaRb)- or -(CRaRb)-CH₂- or -CH₂-(CRaRb)-CH₂-, or - (CH₂)₂-(CRaRb)- or -(CRaRb)-(CH₂)₂- wherein n is a number selected from 1**,** 2 or 3 and Ra and Rb are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino or halogen;
Y is selected from -O-, -S-, -NRa- or -C(O)-, wherein Ra is as previously defined;
W is a group comprising at least a group selected from substituted or unsubstituted aryl, substituted or usubstituted heterocyclyl, substituted or unsubstituted alkenyl;
or a salt, complex or solvate thereof;
already include in their structure the quaternary C8a of TTX or analogues thereof

Further, in contradiction to previously disclosed synthesis of TTX and analogues thereof (see Figure I), by incorporation of a guanidine (or similar) residue to a compound of formula IIa it is possible to easily form the guanidine ring prior to the formation of the orthoester (Figure II):

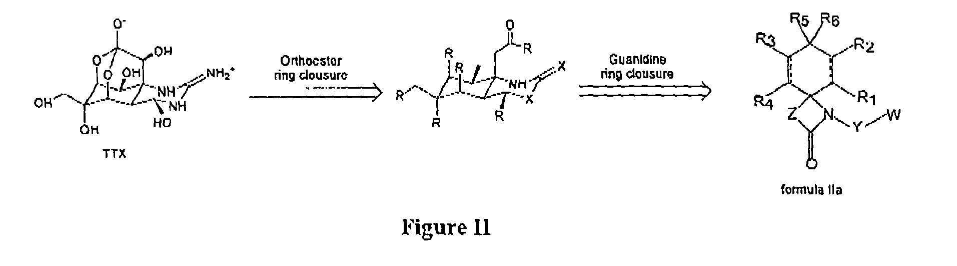

Surprisingly, through this novel strategy TTX and analogues thereof are obtained in overall good yield, in fewer steps and using cheap and readily available materials. Therefore, an aspect of the present invention is a process for the synthesis of a compound of formula 1: 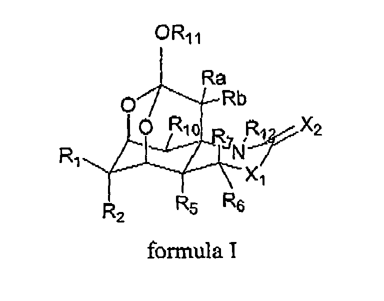 wherein
R₁ and R₂ together are =O or alkenyl; or
R₂ is selected from H, OH or OPr, and R₁ is selected from hydrogen, cyano, substituted or unsubstituted alkyl, alkyl-OPr, substituted or unsubstituted cycloalkyl, substituted or unsubstituted alkenyl, substituted or unsubstituted alkinyl, substituted or unsubstituted aryl, substituted or unsubstituted heterocyclyl;
R₅ is selected from H, OH, OPr;
R₆ and R₇ together are -O or =NPm; or R₆ and R₇ are each independently selected from H, OH, OPr or cyano;
R₁₀ is selected from H, OH, OPr or =O;
R₁₁ is H, Pr or a negative charge;
R₁₂ is H or Prn;
X₁ is selected from NPm, NH, O or S;
X₂ is selected from NPm, NH, ⊕NH₂, O or S;
Ra and Rb are each independently selected from H, OH, OPr or substituted or unsubstituted alkyl, substituted or unsubstituted amino or halogen;
Pr is a hydroxyl protecting group;
Prn is a amino protecting group;
or its salts or tautomers,
comprising the step of forming a orthoester from a compound of formula VII: 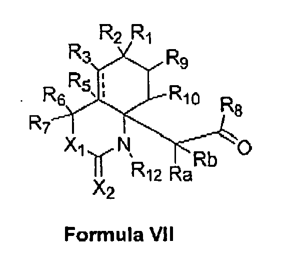 wherein
R₁ and R₂ together are -O or alkenyl; or
R₂ is selected from H, OH or OPr, and R₁ is selected from hydrogen, cyano, substituted or unsubstituted alkyl, substituted or unsubstituted cycloalkyl, substituted or unsubstituted alkenyl, substituted or unsubstituted alkinyl, substituted or unsubstituted aryl, substituted or unsubstituted heterocyclyl;
R₆ and R₇ together are =O or =NPrn; or R₆ and R₇ are each independently selected from H, OH, OPr or cyano;
R₈ is H, OH, ester or amide;
R₉ and R₁₀ are each independently selected from H, OH, OPr or =O;
R₃ is selected from H, OH, OPr or =O,
if the bond between C5 and C4a is a single bond, R₅ is selected from H, OH or OPr; or R₃ and R₅ together are -O-, forming and epoxide ring;
R₁₂ is H or Prn;
Ra and Rb are each independently selected from H, OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino or halogen;
Pr is a hydroxyl protecting group;
Pm is a amino protecting group;
X₁ is selected from NPm, NH, O or S;
X₂ is selected from NPrn, NH, ⊕NH₂, O or S.

A further aspect of the present invention is a process for the synthesis of a compound of formula VII 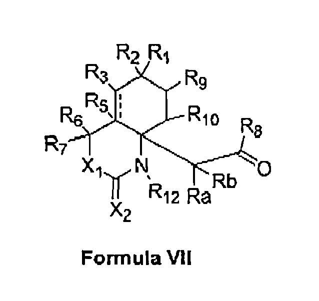 wherein
R₁ and R₂ together are =O or alkenyl; or
R₂ is selected from H, OH or OPr, and R₁ is selected from hydrogen, cyano, substituted or unsubstituted alkyl, substituted or unsubstituted cycloalkyl, substituted or unsubstituted alkenyl, substituted or unsubstituted alkinyl, substituted or unsubstituted aryl, substituted or unsubstituted heterocyclyl;
R₆ and R₇ together are -O or =NPrn; or R₆ and R₇ are each independently selected from H, OH, OPr or cyano;
R₈ is H, OH, ester or amide;
R₉ and R₁₀ are each independently selected from 1-1, OH, OPr or =O;
R₃ is selected from H, OH, OPr or =O,
if the bond between C5 and C4a is a single bond, R₅ is selected from H, OH or OPr; or R₃ and R₅ together are -O-, forming and epoxide ring;
R₁₂ is H or Prn;
Ra and Rb are each independently selected from 11, OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino or halogen;
Pr is a hydroxyl protecting group;
Pm is a amino protecting group;
X₁ is selected from NPrn, NH, O or S;
X₂ is selected from NPrn, NH, ⊕NH₂, O or S;
which comprises opening the 1-aza-2-oxaspiro ring of a compound of formula VI 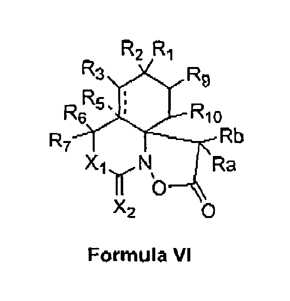 wherein X₁, X₂, R₁, R₂, R₃, R₅, R₆, R₇ R₉, R₁₀, Ra and Rb have the same meaning as in formula VII.

A further aspect of the present invention is a process for the synthesis of a compound of formula I: 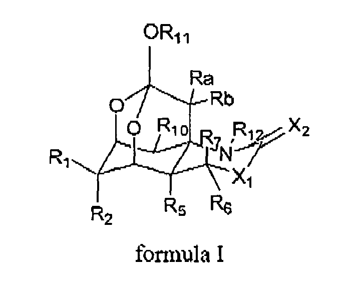 wherein
R₁ and R₂ together arc -O or alkenyl; or
R₂ is selected from H, OH or OPr, and R₁ is selected from hydrogen, cyano, substituted or unsubstituted alkyl, alkyl-OPr, substituted or unsubstituted cycloalkyl, substituted or unsubstituted alkenyl, substituted or unsubstituted alkinyl, substituted or unsubstituted aryl, substituted or unsubstituted heterocyclyl;
R₅ is selected from H, OH, OPr;
R₆ and R₇ together are =O or =NPrn; or R₆ and R₇ are each independently selected from H, OH, OPr or cyano;
R₁₀ is selected from H, OH, OPr or = O;
R₁₁ is H, Pr or a negative charge;
R₁₂ is H or Prn;
X₁ is selected from NPrn, NH, O or S;
X₂ is selected from NPm, NH, ⊙NH₂, O or S;
Ra and Rb are each independently selected from H, OH, OPr or substituted or unsubstituted alkyl, substituted or unsubstituted amino or halogen;
Pr is a hydroxyl protecting group;
Prn is a amino protecting group;
or its salts or tautomers,
comprising the following steps:
a) deprotecting the N-oxide residue of a compound of formula II 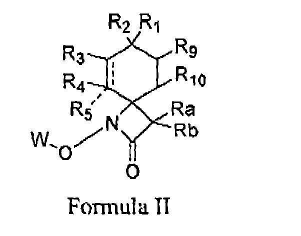 wherein
   R₃ and R₉ are each independently selected from H, OH, OPr or =O;
   R₄ is selected from cyano, substituted or unsubstituted alkyl, OPr, substituted or unsubstituted alkenyl, substituted or unsubstituted alkinyl;
   if the bond between C5 and C4a is a single bond, R₅ is selected from H, OH or OPr; or R₄ and R₅ together are =O;
   or R₃ and R₅ or R₃ and R₂ together are -O-, forming and epoxide ring;
   W is a group comprising at least a group selected from substituted alkyl, substituted or unsubstituted aryl, substituted or usubstitutcd heterocyclyl, substituted or unsubstituted alkenyl;
   R₁, R₂, R₁₀, Ra, Rb and Pr have the same meaning as in Formula I;
   followed by the introduction of a residue of formula III 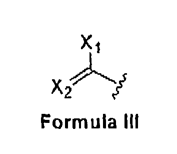 wherein
   X₁ is selected from NHPrn, NH₂, N(Prn)₂, wherein Prn can be the same or different,
   OH, OPr, SH or SPrs;
   X₂ is selected from NPrn, NH, O or S;
   Prs is a sulfur protecting group;
   Pr and Prn have the same meaning as in formula 1;
   to give a compound of formula IV 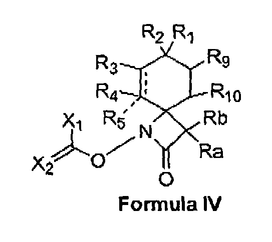 wherein
   R₁, R₂, R₃, R₄, R₅, R₉, R₁₀, Ra and Rb have the same meaning as in formula II;
   X₁ and X₂ have the same meaning as in formula III;
b) closing the ring between X₁ and either R₄ or C4a of the compound of formula IV to give a compound of formula V 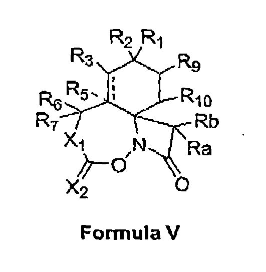 wherein
   X₁ is selected from NPrn, NH, O or S;
   X₂ is selected from NPrn, NH, ⊕NH₂, O or S;
   R₁, R₂, R₃. R₅, R₉, R₁₀, Ra and Rb have the same meaning as in formula II;
   R₆ and R₇ have the same meaning as in formula I;
c) if necessary, subjecting the compound of formula V to a rearrangement to give a compound of formula VI 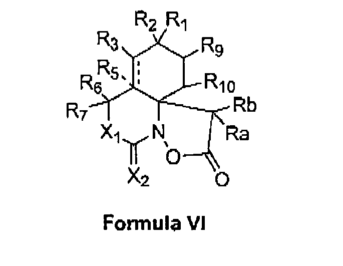 wherein
   R₁, R₂, R₃, R₅, R₆, R₇, R₉, R₁₀, X₁, X₂, Ra and Rb have the same meaning as in formula V;
d) opening the 1-aza-2-oxaspiro residue of the compound of formula VI to give a compound of formula VII. 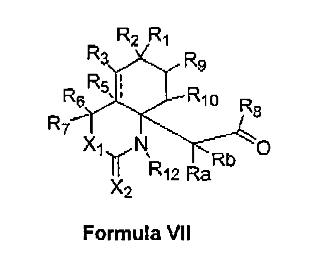 wherein
   R₁ and R₂ together are =O or alkenyl; or
   R₂ is selected from H, OH or OPr, and R₁ is selected from hydrogen, cyano, substituted or unsubstituted alkyl, substituted or unsubstituted cycloalkyl, substituted or unsubstituted alkenyl, substituted or unsubstituted alkinyl, substituted or unsubstituted aryl, substituted or unsubstituted heterocyclyl;
   R₆ and R₇ together are =O or =NPrn; or R₆ and R₇ are each independently selected from H, OH, OPr or cyano;
   R₈ is H, OH, ester or amide;
   R₉ and R₁₀ are each independently selected from H, OH, OPr or =O;
   R₃ is selected from H, OH, OPr or =O,
   if the bond between C5 and C4a is a single bond, R₅ is selected from H, OH or OPr; or R₃ and R₅ together arc -O-, forming and epoxide ring;
   R₁₂ is H or Prn;
   Ra and Rb are each independently selected from 11, OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino or halogen;
   Pr is a hydroxyl protecting group;
   Prn is a amino protecting group;
   X₁ is selected from NPrn, NH, O or S;
   X₂ is selected from NPm, NH, ⊙NH₂, O or S; and
e) formation of the orthoester to give a compound of formula I from a compound of formula VII.

It is to be understood that each one of the steps a) to e) as defined in the method above are in themselves also an aspect of the invention.

According to one embodiment of the invention, the formation of the compound of formula 1 is carried out in two steps wherein
d₁) the compound of formula VII is treated with acid to give a compound of formula Villa 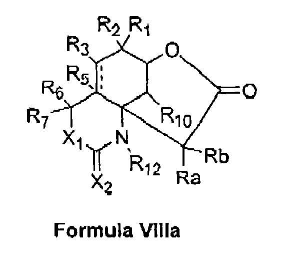 wherein
   R₁, R₂, R₃, R₅, R₆, R₇, R₁₀, R₁₂, X₁, X₂, Ra, Rb, Pr and Prn have the same meaning as in formula VII, if R₉ is OH or OPr; or
d₂) the compound of formula VII is treated with acid to give a compound of formula VIIIb 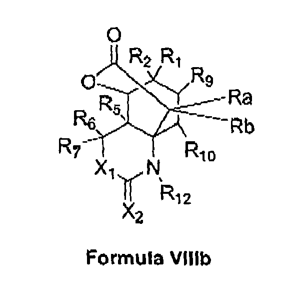 wherein
   R₁, R₂, R₅, R₆, R₇, R₉, R₁₀, R₁₂ X₁, X₂, Ra, Rb, Pr and Pm have the same meaning as in formula VII if R₃ is OH or OPr.

Further aspects of the invention are to provide compounds of formula IV, V, VI and VII as defined above.

A further aspect of the invention is directed to the use of a compound of formula II as defined above, or of a compound as defined in claim 1 of patent application PCT/EP2005/005149, in the synthesis of Tetrodotoxin or analogues thereof.

### DETAILED DESCRIPTION OF THE INVENTION

In studying the structure of compounds obtained in our previous patent applications (EP 04076477.1 and PCT/EP2005/005149) it was realized that the quaternary centre at carbon 8a of TTX had been built. Taking advantage of this it has now been devised the synthesis of TTX and analogues thereof.

In our copending application PCT/FP2005/005146 we described a process for producing a spirolactam compound having the following formula 4 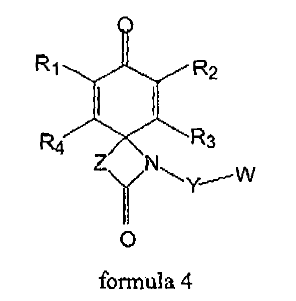 wherein R₁ to R₄ are independently selected from H, halogen, protected or unprotected hydroxy, protected or unprotected silyloxy, substituted or unsubstituted alkyl or cycloalkyl, substituted or unsubstituted alkoxy or aryloxy, substituted or unsubstituted aryl, substituted or unsubstituted heterocyclyl, nitro, substituted or unsubstituted amino, mercapto, substituted or unsubstituted arylthio or alkylthio;
Z is -(CRaRb)ₙ- or -CH₂-(CRaRb)- or -(CRaRb)-CH₂- or -CH₂-(CRaRb)-CH₂-, or - (CH₂)₂-(CRaRb)- or -(CRaRb)-(CH₂)₂- wherein n is a number selected from 1, 2, 3 and Ra and Rb are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino, or halogen;
Y is selected from -O-, -S-, -NRa- or -C(O)-, wherein Ra is as previously defined; and
W is a group with sufficient electronic density to stabilize the compound through π (pi) interactions with the benzodienone moiety such as a group selected from substituted or unsubstituted arylalkyl, substituted or usubstituted heterocyclylalkyl, substituted or unsubstituted alkenyl;
comprising the steps of reacting a compound of formula 3 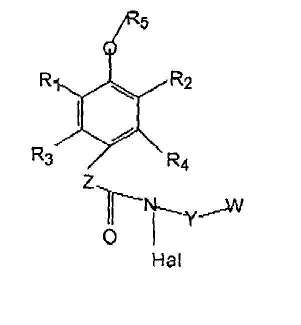 wherein R₅ is hydrogen or susbtituted or unsubstituted alkyl and Hal is F, Cl, Br, 1 or -SO₂CF₃;
with an N-acylnitrenium ion forming agent such as a silver salt to produce the spirolactam compound.
The compound of formula 3 is prepared by reacting a Weinreb-type amide compound of formula: 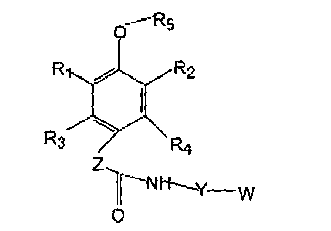 with a a halogenating agent; preferably an agent selected from alkyl hypochlorite, alkyl hypobromite, sodium bromite, sodium hypochlorite, benzyltrimethylammonium trihalide, N-halosuccinimide, N-halophthalimide, or phenyliodine (III) bis(trifluoroacetate) (PIFA).

The process to obtain the starting materials (compounds of formula II) of the present invention arc described in our copending application PCT/EP2005/005149, and involve starting from a compound of formula 4 above and then applying a basic set of reactions selected from:
a) Hydroxilation dihydroxylation or ketohydroxylation: using mild (such as OsO₄/ N oxide amine) or strong systems (such as RuCl₃) depending on the position to be hydroxylated. Alternative systems are also envisaged.
b) Nucleophilic attack at the carbonyl group: for example with a carbanion on a sp, sp2 or sp3 C, the carbanion can be prepared or generated in situ; or with an hydride. The Nucleophilic attack allows the introduction of new functionalities, new carbonated structures and also epoxides or double bonds, depending on the reagents used.
c) Hydroxyl inversion : for example through epimerization or inversion, for example in Mitsunobu conditions.
d) Hydroxyl or carbonyl protection: using the same or different protecting groups in conditions as explained above.
e) Allylic rearrangements: allows migration of a double bond.

Although each of these procedures are well known and the appropriate reagents can be selected by the person skilled in the art of organic synthesis, their application to our structures gives new compounds and unexpected results in terms of reactivity and selectivity.

Thus the different intermediate compounds of the invention and the starting materials can be prepared using a basic set of simple reactions that allow the protection, functionalisation of the different positions in a very stereospecific manner. These procedures will be explained below. Throughout the description and claims the configurations given arc only relative configurations. Enantiomeric compounds of those described may be prepared using starting materials or reagents when needed which have the opposite optical rotation.

The starting materials for the processes of the present invention are the compounds of formula II. As mentioned before, synthetic approaches to these compounds are disclosed in our PCT/EP2005/005149 and PGT/EP2005/005146 which are incorporated herein by reference in their entirety. The compounds of formula II can be readily prepared with the desired configuration and functionality, from commercial starting materials, and using a basic set of reactions.

Compounds of formula II are excellent starting materials for the synthesis of TTX derivatives. As shown in our previous patent application, compounds of formula II with different patterns of substitution may be readily prepared. This versatility allows the preparation of different analogues of TTX following the same basic steps as disclosed in the present patent application.

According to one embodiment, compounds of formula II are compounds of formula IIa: 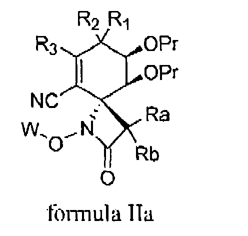 wherein R₁, R₂, R₃, Ra, Rb, W and Pr have the same meaning as in formula II.

According to a further embodiment, compounds of formula II are compounds of formula IIb: 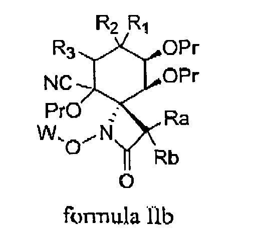 wherein R₁, R₂, R₃, Ra, Rb, W and Pr have the same meaning as in formula II.

According to a further embodiment, compounds of formula II arc compounds of formula IIe: 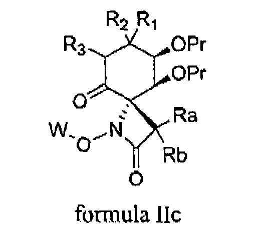 wherein R₁, R₂, R₃, Ra, Rb, W and Pr have the same meaning as in formula II.

According to a further embodiment, compounds of formula II arc compounds of formula IId 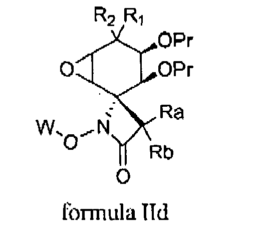 wherein R₁, R₂, R₃, Ra, Rb, W and Pr have the same meaning as in formula II.

According to a further embodiment, compounds of formula II are compounds of formula IIe 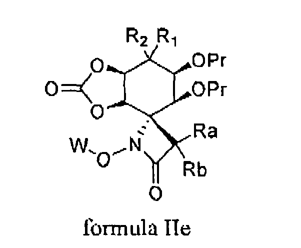 wherein R₁, R₂, R₃, Ra, Rb, W and Pr have the same meaning as in formula a II.

A compound of formula IIa-IId may itself be transformed in a different compound of formula IIa-IId. For example, compounds of formula IIc can easily be transformed into compounds of formula IIa or IIb.

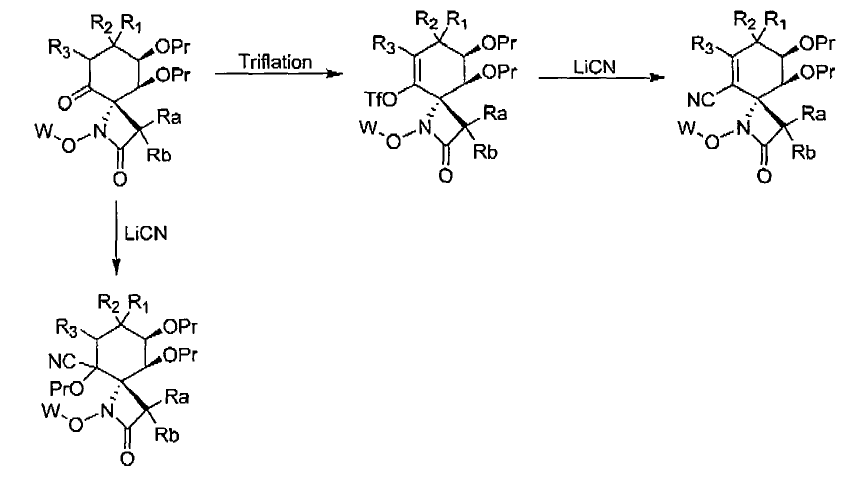

The reaction conditions for the above mentioned reactions are known to the skilled person. Triflation is a standard reaction which may be performed, for example, following conditions described in Tetrahedron Lett. 1984, 25, 595-598. Introduction of a cyano group may be performed by nucleophilic substitution following, for example, the conditions described in Can. J. Chem. 1993, 71, 1867-1872.

Having already the quaternary centre at C8a, there are many routes which can give a compound of formula IV by removing the residue W and incorporating a residue of formula III. W can be considered an O protecting group, and therefore any procedures adequate to remove protecting groups of the nature of W can be used. For example, for a compound of formula IIc, wherein W is a benzyl group the following sequence yields the desired compound of formula IV:

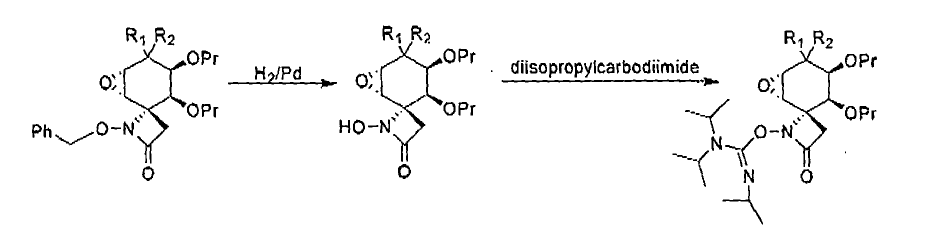

Different kind of reagents can be used to introduce the group of formula III. For example thioureas, carbodiimides, isocyanates or thioisocyanates can be used. Other alternatives will be apparent to the person skilled in the art.

Alternatively, it is also possible to prepare a compound of formula IV, using the methodologies described in the two mentioned patent applications, wherein the W group is in fact a group of formula III. In other words, the group of formula III can be introduced as early in the synthesis as desired, saving in some cases one step in the synthesis,

The residue of formula III may incorporate the guanidine residue of TTX and analogues thereof (positions 1, 2 and 3). In a preferred embodiment according to the present invention, X₁ is selected from N(Prn)₂, wherein Prn groups can be the same or different, NH or NHPrn, and X₂ is NPm.

Compounds of formula IV already have the atoms necessary to build the basic skeleton of TTX and analogues thereof. According to one embodiment, compounds of formula IV are compounds of formula IVa 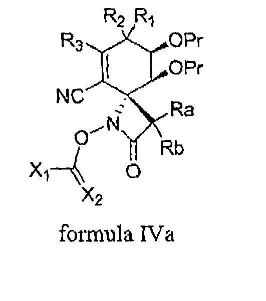 wherein R₁, R₂, R₆, R₇, Ra, Rb, X₁, X₂ and OPr are as defined in formula IV.

According to one embodiment, compounds of formula IV arc compounds of formula IVb 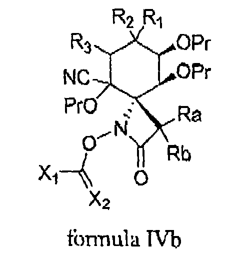 wherein R₁, R₂, R₆, R₇, Ra, Rb, X₁, X₂ and OPr are as defined in formula IV.

According to another embodiment, compounds of formula IV are compounds of formula IVc 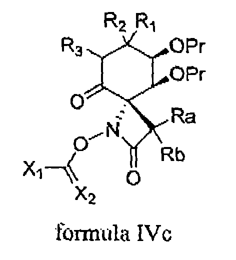 wherein R₁, R₂, R₆, R₇, Ra, Rb, X₁, X₂ and OPr are as defined in formula IV.

According to another embodiment, compounds of formula IV are compounds of formula IVd 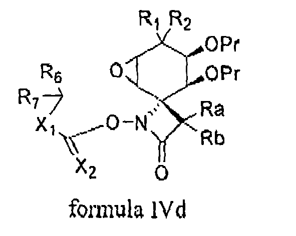 wherein R₁, R₂, R₆, R₇, Ra, Rb, X₁, X₂ and OPr are as defined in formula IV.

According to another embodiment, compounds of formula IV are compounds of formula IVe 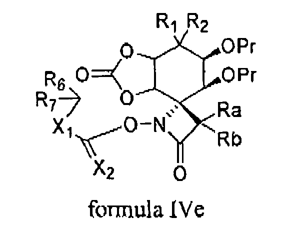 wherein R₁, R₂, R₆, R₇, Ra, Rb, X₁, X₂ and OPr are as defined in formula IV.

In the same way as for compounds of formula II, the compound of formula IVa-IVd may itself be transformed in a different compound of formula IVa-IVd. A particular example is shown below

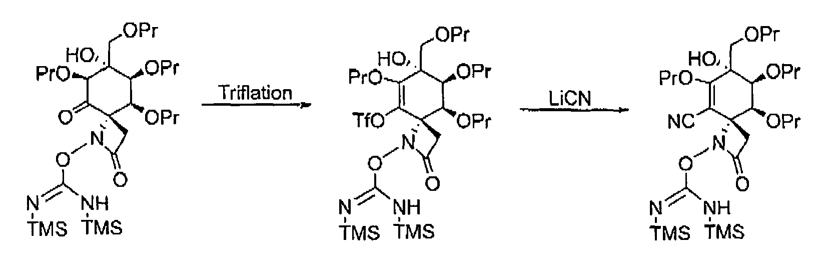

The ring closure of the guanidine ring is performed through simple reactions depending on the compounds of formula II selected as precursors. For example, where R₄ is CN (compounds according to formula IVa or lVb) and X₁ is N(Prn)₂, wherein Prn is the same or different, NH₂ or NHPrn, acid treatment yields the desired compound of formula V through a rearrangement of the rings to give the more stable 2-iminotetrahydropirimidin-4(1*H*)-one. For example:

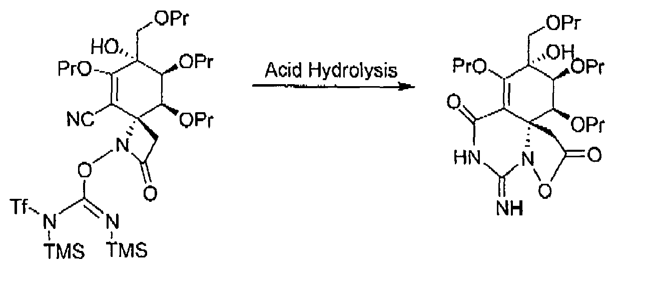

Examples of acids which can be used, are triflic acid, HCl, sulphuric acid, etc.

However, it is also possible to perform the stepwise synthesis of compounds of formula VI by mild acid treatment of compounds of formula IV, isolating the resulting compounds of formula V having a 7-imino-1,2,6-oxadiazepan-5-one ring, which are further treated under stronger acid conditions or heat to give compounds of formula VI.

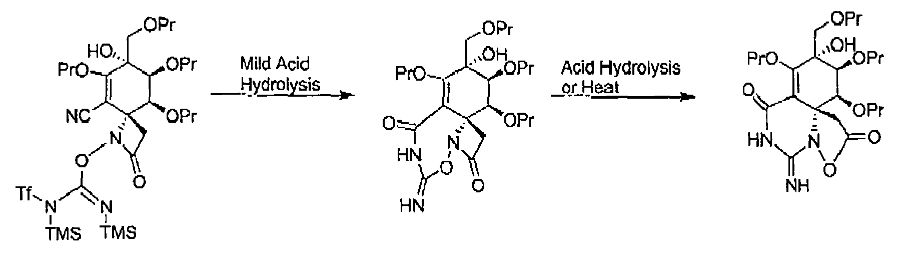

In the case of compounds IVd and IVe ring closure is achieved under different conditions and C4 is provided by X₁. Treatment of a compound of formula IVd or IVc with a strong base such as LDA or LiHMS, forms an anion which attacks the cpoxidc or the carbonate.

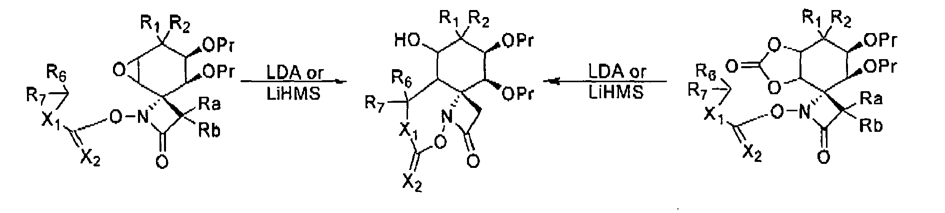

In a variant of the invention a precursor of the group of formula III can be first linked to the position 4a or 4 and then to the N of the lactame ring.

In a further variant of the invention the N-hydroxy moiety of compounds of formula II may be completely deprotected under reductive conditions to the lactam and the precursor of the group of formula III linked directly to the nitrogen of the lactam.

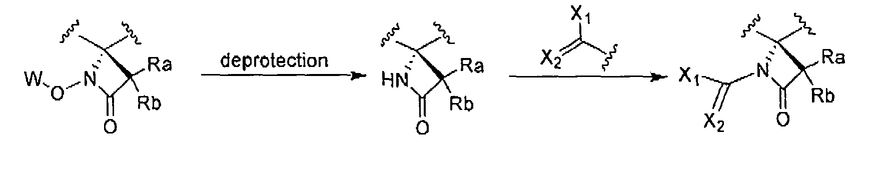

In a further variant of the invention the nitrogen in position 1 of the spirolactam may be protected as a hydrazine group 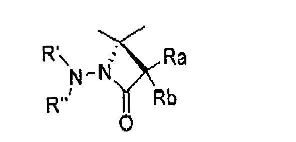 wherein, preferably, R' and R" are each indepently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino or halogen. The N-N bond is removed afterwards under reductive conditions.

It has also been observed that treatment of a compound of formula IV under acidic conditions, when R₂ is OH or OPr and R₄ is cyano, both being in relative cis position, it is possible to obtain the lactone 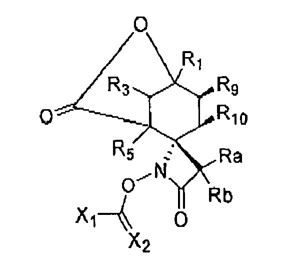 which itself undergoes ring closure to give a compound of formula V or VI.

From the compound of formula VI, further ring opening of the 1-aza-2-oxaspiro residue and protection of the nitrogen in position 1 can be readily carried out. In a preferred embodiment according to the present invention, said ring opening is performed via catalytic hydrogenation.

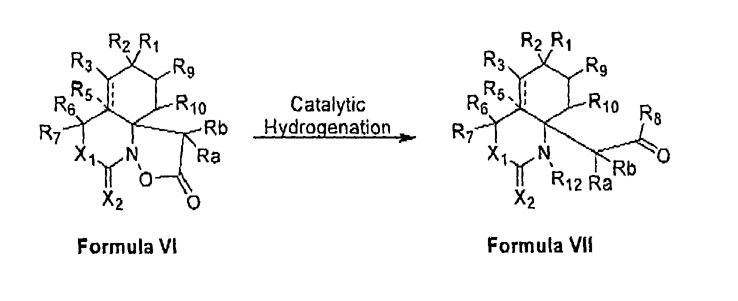

Compounds of formula VII are ready for orthoester bridge closure, which may be performed via a stepwise sequence, first forming a lactone and then the orthoester, or by direct formation of the orthoester in a single step.

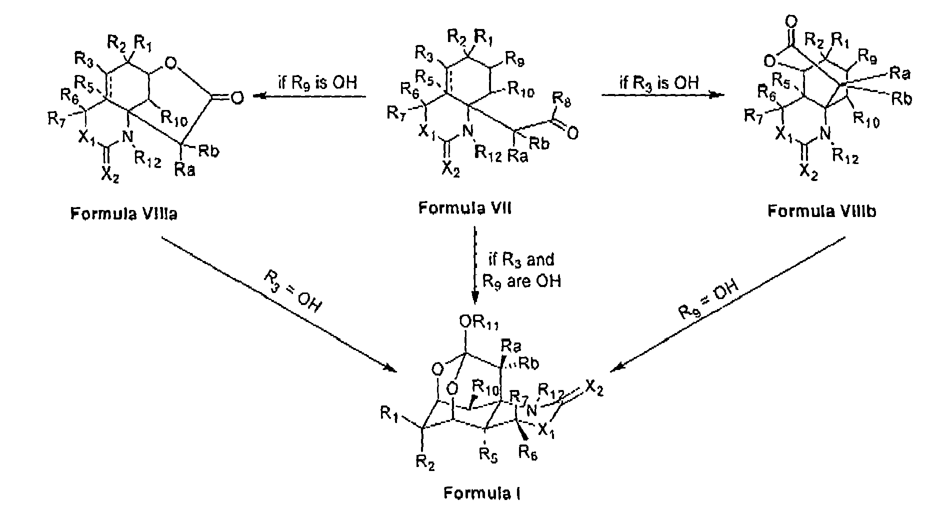

Conditions under which orthoester ring closure has been performed arc known to the skilled person and generally involve acid treatment. According to one embodiment of the present invention, a compound of formula VII wherein R₃, R₈ and R₉ are OH is treated acid treatment, preferably HCl, fellowed by peracetylation. The resulting acetylated compound is treated with hydride, followed by triethylamine in water-methanol, to give TTX (see Isobe, M.; Urabe, D.; Nishikawa, T. Angew. Chem. Int. Ed. 2004, 43, 4782-4785). In a further embodiment, persylilation me be carried out instead of peracetylation.

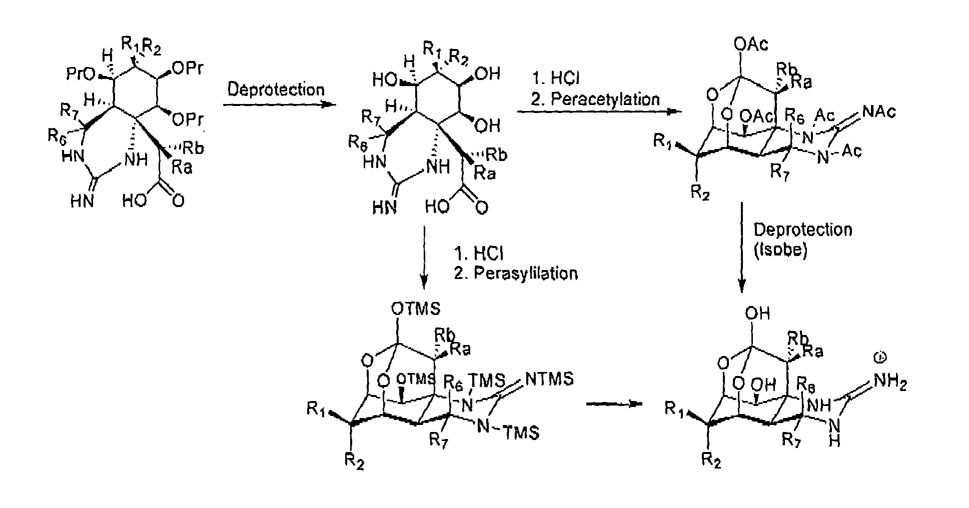

In a preferred embodiment, R₃ and R₉ are OPr, wherein OPr is a silyl protecting group, and R₈ is OH. This compounds achieve deprotection and formation of the orthoester in a single step by treatment with acid, preferably HCl.

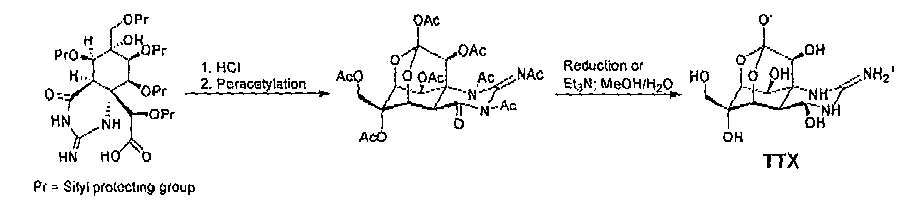

Further, this protection strategy is specially useful when other hydroxyl groups in the molecule are protected with benzoate, since both, being different protecting groups, are removed under the same conditions in one step.

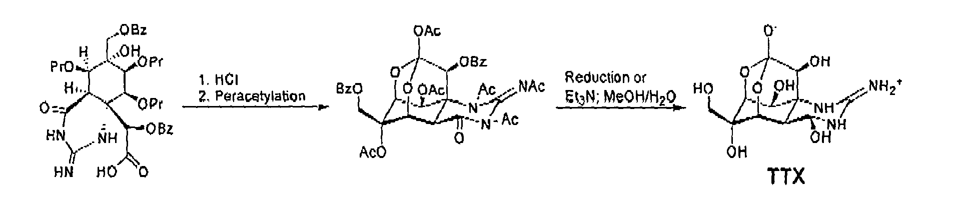

In a further embodiment, compounds of formula VII wherein R₃ and R₅ together are -O-, forming and epoxide ring, are also treated under acidic conditions followed by persylilation or peracetylation. As in the previous embodiment, although the reaction may be carried out in three steps (deprotection, acid treatment, persylilation or peracetylation), an adequate selection of protecting groups yields TTX or an analogue thereof in two steps.

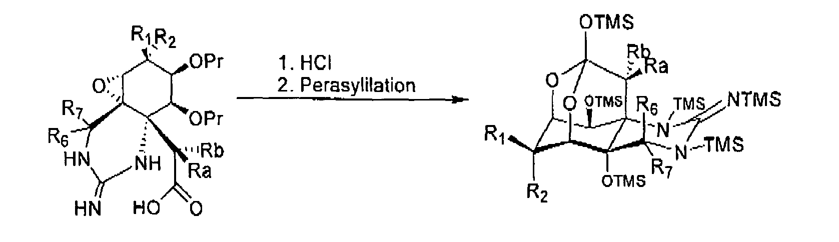

There are a number of reactions which have been performed in different stages of the synthesis. One example is protection and deprotection reactions which have been performed following known conditions to the skilled person. Examples of protecting groups can be found in reference books such as Greene and Wuts' "Protective Groups in Organic Synthesis", John Wiley & Sons, Inc., New York, 1999. Adequate protection strategies can be selected on the different hydroxy or amino groups in order to simplify and control the different steps of the processes.

Also, hydrogenation, when necessary, of the C5-C4a bond has been performed in compounds of formula IV, V, VI, VII or VIIIa. The election of the most suitable stage for hydronegation depends mainly on the protecting groups present in the molecule.

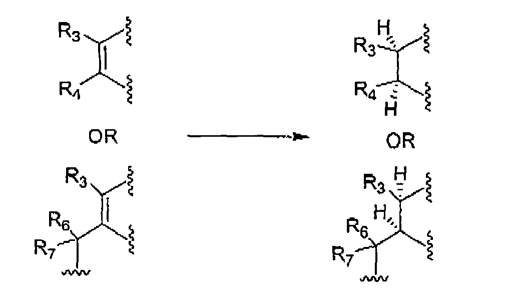

As disclosed in the previous scheme, hydrogenation usually takes place from the less hindered face. For example, in the case of a compound of formula IVa, catalytic hydrogenation proceeds with almost complete stereoselectivity when R₃, R₉ and R₁₀ are OPr. We believe this is due to steric hinderance between R₉ and Ra and Rb.

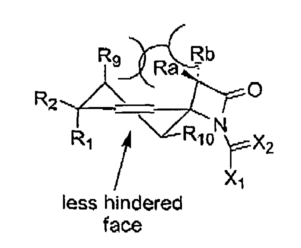

On the other hand, in some sequences it has been necessary to perform the opposite reaction and introduce a double bond between C4a and C5. This can he readily achieved from a compound of formula V, VI, VII or VIIIa, wherein R₃ is OH, by oxidation to form the ketone in C5, and subsequent enolization-protection.

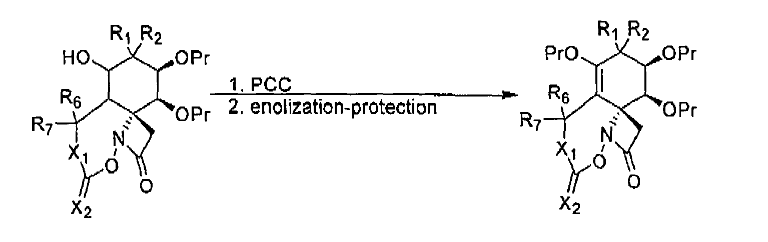

Having a double bond between C4a and C5 an epoxide ring has been introduced through conventional conditions in compounds of formula 11, IV, V, VI and VII.

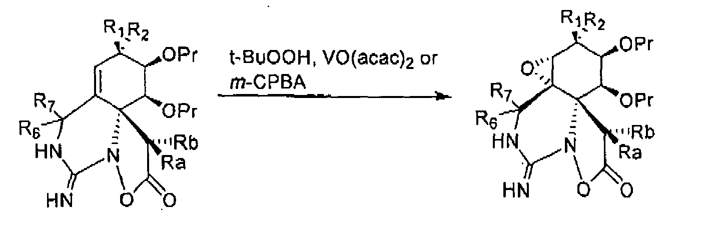

Also, if it is necessary to introduce the hydroxyl group at C₉, hydroxylation α to a carbonyl group is readily achieved using conditions known to the skilled person. For example, formation of the corresponding enolate followed by addition of oxaziridine to a compound of formula II-VIII results is the introduction of the hydroxyl group (see J.Org.Chem. 1992, 57,6387-6389). The hydroxyl group can then be protected with any desired hydroxyl protecting group. This reaction can also be performed at different stages of the synthesis. Further, compounds of formula II may already comprise the hydroxyl group (or a suitable precursor) at C9.

When the previous hydroxylation at C9 arc carried out, two isomers are possible, and the one corresponding to the less hindered face of the molecule is obtained in greater yield. If the hydroxyl with the desired configuration at C9 is obtained, protection can in some cases be necessary in order to avoid the formation of 4-dehydroxy by-products.

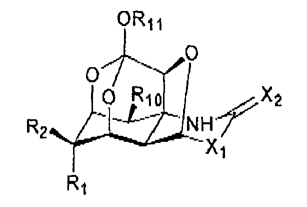

In other cases, the TTX derivative with the opposite configuration at C9 can be obtained as the mayor product. This derivatives are also useful because they do not form the 4-dehydroxy by-products above mentioned.

However, epimerization of different hydroxyl functional groups, including at C9, throughout the synthesis can be carried out under Mitsonobu conditions (Mitsunobu, 0-; Synthesis, 1, 1981**)**

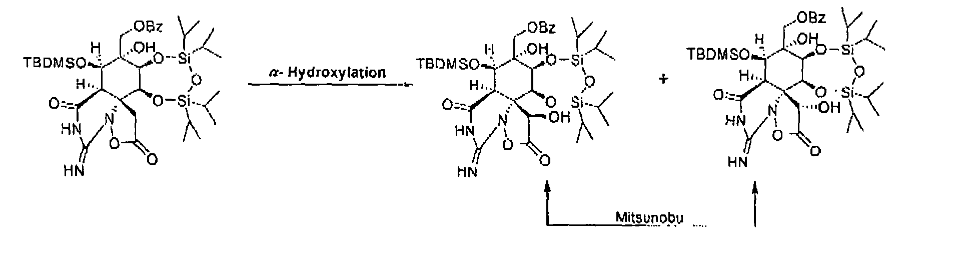

In our previous patent applications EP 04076477.1 and PCT/EP2005/005149 we devised different reaction sequences in order to arrive to the correct structure of R₁ and R₂. The same reaction sequences can also be successfully applied to compounds of formula 1-VIIIb. For example, introduction of C11 in a compound of formula VI having ketone at C6 can be performed in three different ways

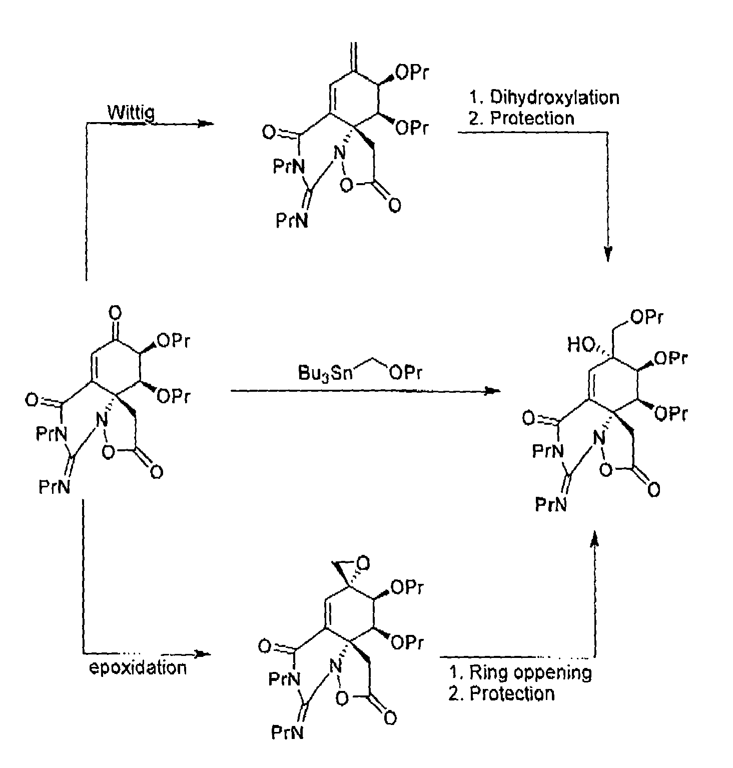

In some reaction sequences it is necessary to remove R₅ (for example, wherein R₅ is OPr) in order to arrive to the correct structure of TTX. This can be done in compounds of formula 1, 11, IV, V, VI, VII or VIII by treatment with a base. For example, an -OTMS on C4a in a compound of formula I is readily removed by treatment with NaHMDS.

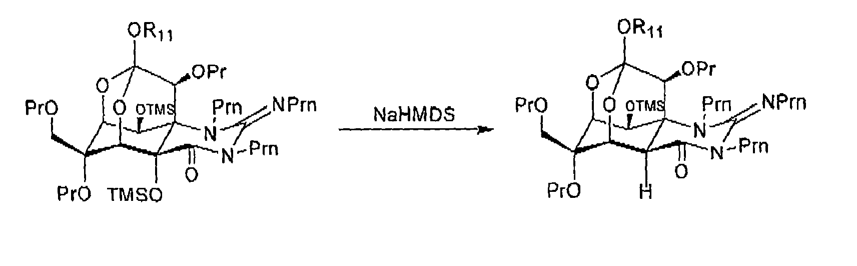

According to one embodiment, compounds of formula V are compounds of formula Va 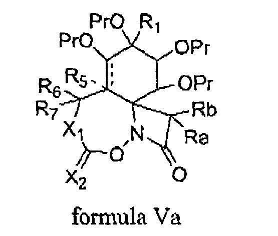 wherein R₁, R₅, R₆, R₇, Ra, Rb, Pr, X₁ and X₂ are as defined in formula V.

According to a further embodiment, compounds of formula VI are compounds of formula VIa 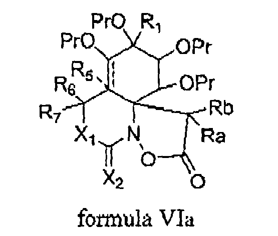 wherein R₁, R₅, R₆, R₇, Ra, Rb, Pr, X₁ and X₂ are as defined in formula VI.

According to a further embodiment, compounds of formula VII are compounds of formula VIIa 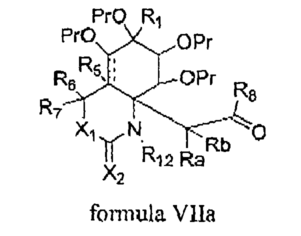 wherein R₁, R₅, R₆, R₇, R_{12;} Ra, Rb, Pr, X₁ and X₂ are as defined in formula VII.

From the above, it is evident to the skilled person the synthesis of different analogues of TTX. For example, the analogues described by Sato (scc figure 1, page 7497 in Sato, K.; Akai, S.; Sugita, N.; Ohsawa, T.; Kogure, T.; Shoji, H.; Yoshimura, J. J. Org. Chem, 2005, 70, 7496-7504) are readily prepared following the synthesis disclosed in the present patent application starting from a suitable compound of formula II. For example, 8, 11-dideoxy TTX is readily available from a compound of formula II, IV, V, VI, VII, Vllla or VIIIa wherein R₁, is a methyl group and R₁₀ is hydrogen. Also new useful analogues which have not been described so far, can also be prepared using the structures and set of reactions of the present invention. Different nor or deoxy -derivatives are obtained by putting into practice the teaching of the present invention. One example is 4-nor TTX, which be obtained from a compound of formula IVc.

Further embodiments of the compounds of the invention are described below and in the examples. It is evident for the skilled person that many variations and configurations arc possible and can be obtained at will depending on the compounds selected as starting materials, the relative configurations of the functional groups present, the presence or not of chiral centers, and on the combination of reactions that are applied. The present invention encompasses all such variations and possibilities.

In the above definition of compounds of formula I-VIIIb and in the description the following terms have the meaning indicated:
"Alkyl" refers to a straight or branched hydrocarbon chain radical consisting of carbon and hydrogen atoms, containing no saturation, having 1-12, preferably one to eight carbon atoms, and which is attached to the rest of the molecule by a single bond, e. g., methyl, ethyl, n-propyl, i-propyl, n-butyl, t-butyl, n-pentyl, etc. Alkyl radicals may be optionally substituted by one or more substituents such as halo, hydroxy, alkoxy, OPr, OBn, OBz, carboxy, cyano, carbonyl, acyl, alkoxycarbonyl, amino, imino, nitro, mercapto and alkylthio.
"Alkoxy" refers to a radical of the formula -ORa where Ra is an alkyl radical as defined above, e. g-, methoxy, ethoxy, propoxy, etc. "Aryloxy" refers to a radical of formula -ORb wherein Rb is an aryl radical as defined below. "Amino" refers to a radical of the formula-NH₂, -NHRa, -NRaRb.
"Aryl" refers to an aromatic hydrocarbon radical such as phenyl, naphthyl or anthracyl. The aryl radical may be optionally substituted by one or more substituents such as hydroxy, mercapto, halo, alkyl, phenyl, alkoxy, haloalkyl, nitro, cyano, dialkylamino, aminoalkyl, acyl and alkoxycarbonyl, as defined herein.
"Aralkyl" refers to an aryl group linked to an alkyl group such as benzyl and phenethyl.
"Cycloalkyl" refers to a saturated carbocyclic ring having from 3 to 8 carbon atoms.
"Heterocyclyl" refers to a stable 3- to 15- membered ring which consists of carbon atoms and from one to five heteroatoms selected from the group consisting of nitrogen, oxygen, and sulphur, preferably a 4-to 8-membered ring with one or more heteroatoms, more preferably a 5-or 6-membered ring with one or more heteroatoms. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quaternized; and the heterocyclyl radical may be partially or fully saturated or aromatic. Examples of such heterocycles include, but arc not limited to, azepines, benzimidaxole, benzothiazole, furan, isothiazole, imidazole, indolc, piperidine, piperazine, purine, quinoline, thiadiazole, tetrahydrofuran.
"Hydroxyl protecting group" refers to a group that blocks the OH function for further reactions and can be removed under controlled conditions. The hydroxyl protecting groups are well known in the art, representative protecting groups are silyl ethers such as trimethylsilyl ether, triethylsilyl ether, tert-butyldimethylsilyl ether, tert-butyldiphenylsilyl ether, tri-isopropylsilyl ether, diethylisopropylsilyl ether, thexyldimethylsilyl ether, triphenylsilyl ether, di-tert-butylmethylsilyl ether; alkyl ethers such as methyl ether, tert-butyl ether, benzyl ether, p-methoxybenzyl ether , 3,4-dimethoxybenzyl ether, trityl ether; allyl ether; alkoxymethyl ether such as methoxymethyl ether, 2-methoxyethoxymethyl ether, benzyloxymethyl ether, p-methoxybenzyloxymethyl ether, 2-(trimethylsilyl)ethoxymethyl ether; tetrahydropyranyl and related ethers; methylthiomethyl ether; Esters such as acetate ester, benzoate ester; pivalate ester; methoxyacetate ester; chloroacetate ester; levulinate ester; Carbonates such as benzyl carbonate, p-nitrobenzyl carbonate, tert-butyl carbonate, 2,2,2-trichloroethyl carbonate, 2-(trimethylsilyl)ethyl carbonate, allyl carbonate; and sulphates such as SO₃py. In some cases activating groups such as triflatc are also included as protecting groups. Additional examples of hydroxyl protecting groups can be found in reference books such as Greene and Wuts' "Protective Groups in Organic Synthesis", John Wilcy & Sons, Inc., New York, 1999.
"Amino protecting group" refers to a group that blocks the NH₂ function for further reactions and can be removed under controlled conditions. The amino protecting groups are well known in the art, representative protecting groups are carbamates and amides such as substituted or unsubstituted or substituted acetates. Also different alkyl moeties may serve as amino protecting groups. Said alkyl groups may optionally be substituted by one or more substituents such as halo, hydroxy, alkoxy, alkyloxymethyl ethers, carboxy, cyano, carbonyl, acyl, alkoxycarbonyl, amino, nitro, mercapto and alkylthio. Additional examples of amino protecting groups can be found in reference books such as Greene and Wuts' "Protective Groups in Organic Synthesis", John Wiley & Sons, Inc., New York, 1999.
"Sulfur protecting group" refers to a group that blocks the SH function for further reactions and can be removed under controlled conditions. Examples of sulfur protecting groups can be found in reference books such as Greene and Wuts' "Protective Groups in Organic Synthesis", John Wiley & Sons, Inc., New York, 1999.

References herein to substituted groups in the compounds of the present invention refer to the specified moiety that may be substituted at one or more available positions by one or more suitable groups, e. g., halogen such as fluoro, chloro, bromo and iodo ; cyano; hydroxyl ; nitro ; azido ; alkanoyl such as a C1-6 alkanoyl group such as acyl and the like ; carboxamido ; alkyl groups including those groups having 1 to about 12 carbon atoms or from 1 to about 6 carbon atoms and more preferably 1-3 carbon atoms ; alkenyl and alkynyl groups including groups having one or more unsaturated linkages and from 2 to about 12 carbon or from 2 to about 6 carbon atoms ; alkoxy groups having one or more oxygen linkages and from 1 to about 12 carbon atoms or 1 to about 6 carbon atoms ; aryloxy such as phenoxy ; alkylthio groups including those moieties having one or more thioether linkages and from 1 to about 12 carbon atoms or from 1 to about 6 carbon atoms ; alkylsulfinyl groups including those moieties having one or more suliinyl linkages and from 1 to about 12 carbon atoms or from 1 to about 6 carbon atoms ; alkylsulfonyl groups including those moieties having one or more sulfonyl linkages and from 1 to about 12 carbon atoms or from 1 to about 6 carbon atoms ; aminoalkyl groups such as groups having one or more N atoms and from 1 to about 12 carbon atoms or from 1 to about 6 carbon atoms ; carbocylic aryl having 6 or more carbons, particularly phenyl or naphthyl and aralkyl such as benzyl. Unless otherwise indicated, an optionally substituted group may have a substituent at each substitutable position of the group, and each substitution is independent of the other.

In the preparation of compounds wherein R₉ and R₁₀ are both protected hydroxyl groups, the two hydroxyl groups can be protected with two different protecting groups, first protecting the position C7 and then the position C8.

To introduce protecting groups standard procedures can be used, such as those described in Greene and Wuts' "Protective Groups in Organic Synthesis", John Wiley & Sons, Inc., New York, 1999 or Kocienski, P.J. "Protecting Groups", 3rd Ed.Thieme Chemistry, 2003.

The presence of vicinal hydroxyl groups allows the simultaneous protection of two of them through the use of diol protecting groups if desired. Among the diol protecting groups that can be used we have O,O-acetals such as isopropylidene acetals (acetonides); cyclohexylidene and cyclopentylidene acetals; arylmethylene acetals; methylene acetals; diphenylmethylene acetals; 1,2-diacetals such as dispiroketal (dispoke) derivatives, cyclohexane-1,2-diacetals, butane-2,3-diacetals; silylene derivatives; 1,1,3,3-tetraisopropyldisiloxanylidene derivatives or N,O-acetals. Additional examples of diol protecting groups can be found in reference books such as Greene and Wuts' "Protective Groups in Organic Synthesis", John Wiley & Sons, Inc., New York, 1999. Additionally, borolanes can be formed on the two vicinal hydroxy groups, for example using phenylboric acid.

One example of such a compound with vicinal protection is :

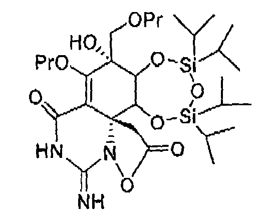

In this case the 7 member ring accommodates the axial-equatorial position of the two OH being protected.

As an alternative, dihydroxylation and protection can be carried out simultaneously: 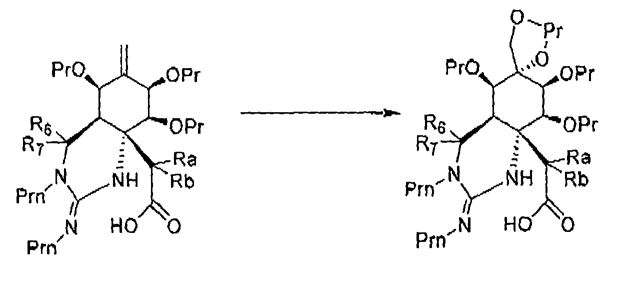 Or, if desired, only one of the hydroxy groups can be selectively protected due to their different nature (primary versus tertiary alcohol):

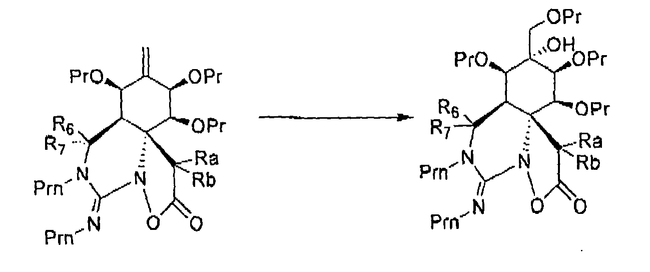

The use of protecting groups of a certain size, or that are linked simultaneously to two different positions can therefore control the stereochemistry of the product.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

The compounds of the invention may be in crystalline form either as free compounds or as solvates (e.g. hydrates) and it is intended that both forms arc within the scope of the present invention. Methods of solvation are generally known within the art.

The invention will be further illustrated by means of examples, which should not be interpreted as limiting the scope of the claims.

### EXAMPLES

### General Methods and Materials.

All reactions described below were carried out under argon atmosphere unless otherwise noted. The solvents used were distilled and dried under argon atmosphere before use. All starting materials were purchased commercially (Aldrich, Fluka and Merck) and used without further purification. Flash Chromatography was executed on columns loaded with 230-400 mesh silica gel Merck. TLC was carried out on silica gel Merck (Kieselgel 60F-254).

Melting points (mp) were determined on a Reichert Microscopic Hot-Stage and arc uncorrected- ¹H and ¹³C NMR spectra were measured on a Varian Gemini-200 and a Varian Inova-300 spectrometer with (CH₃)₄Si as an internal reference and CDCl₃ as solvent unless otherwise noted. Both ¹H and ¹³C NMR spectral data are reported in parts per million (δ) relative to residual sign of the solvent (CDCl₃, 7.26 ppm and 77.0 ppm for ¹H and ¹³C NMR, respectively). ¹H and ¹³C NMR designations arc: s (singlete); s br. (broad singlete); d (doublete); t (triplete); q (quartete); m (multiplete). Infrared (IR) spectra were record on a Perkin-Elmer FT-IR spectrometer. U V spectra were record on a Perkin-Elmer 402 spectrometer. Low-resolution mass (LRMS) spectra were obtained on a Hewlett Packard 5973 MSD spectrometer with a direct inlet system (EI) at 70 eV. Microanalytical data (E.A.) were obtained on a Perkin-Elmer 240C and Heraus CHN-O instruments at the Instrumental Analysis Department of lnstituto de Quimica Orgánica General (C.S.I.C.).

### Example 1:

### Preparation of rac-(4S,6S,7R,8S,9S)-1-Bencyloxy-7-benzoyloxymethyl-6-tert-butyldimethylsyliloxy-7,8,9-trihydroxy-8,9-O-(1,1,3,3-tetraisopropyldisyloxane-1,3-diyl)-1-azaspiro[3.5]nonane-2,5-dione (70)

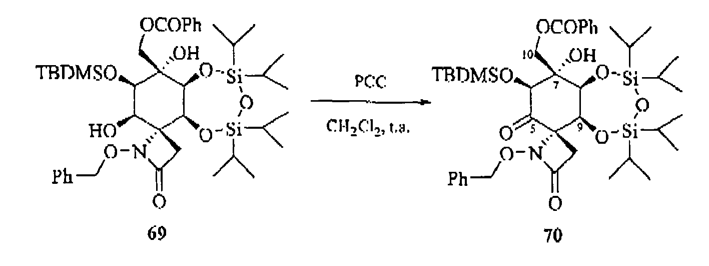

PCC (15 mg, 0,072 mmol, 1.5 eq.) was added to a solution of *rac*-(4*R*,5*S*,6*S*,7*S*,8*R*,9*R*)-1-benzyloxy-7-benzoyloxymethyl-8-*tert*-butyldimethylsilyloxy-5,6,7,9-tetrahydroxy-5,6-*O-*(1,1,3,3-tetraisopropyldisiloxane-1,3-diyl)-1-azaspiror[3.5]nonan-2-one (**69**) (39 mg, 0.048 mmol, 1.0 eq.) in CH₂Cl₂ (0,6 ml) and was stirred for 20 hours at room temperature. After that time, a little amount of Celite was added and the solvent was eliminated under reduced pressure. The product was purified by column chromatography (hexane/AcOEt, 10:1) and *rac*-(4*S*,6*S*,7*R*,8*S*,9*S*)-1-benzyloxy-7-benzoyloxymethyl-6-*tert*-butyldimethylsilyloxy-7,8,9-trihydroxy-8,9-*O*-(1,1,3,3-tetraisopropyldisiloxane-1,3-diyl)-1-azaspiro[3.5]nonane-2,5-dione **(70)** (36 mg. 92% yield) was obtained as a white solid, Also, the starting product **69** (2 mg, 5%) and an isomer of 70 (1mg, 3% yield) was recovered as a white solid.
**P.f**.: 163-164°C.
***R_{f}**=* 0.53 (TLC, hexano/AcOEt, 3:1).
**¹H-NMR** (200MHz, CDCl₃): δ 8.03 (2H, dm, *J =* 6.9 Hz, Bz), 7.64 (1H, tm, *J* = 7.3 Hz, Bz), 7.49 (2H, ddm, *J* = 7.3, 6.9 Hz, Bz), 7.44-7.27 (5H, m, Ph), 5.17 (2H, s, OCH₂Ph), 4.85 (1H, d, *J* = 4.2 Hz, H-9), 4.79 (1H, *part A syst. AB, J =* 11.7 Hz, H-10), 4,57 (1 H, dd, *J* = 4.2, 1.3 Hz, H-8), 4.56 (1H, *part B syt. AB, J =* 11.7 Hz, H-10'), 4.12 (1H, d, *J* = 1.3 Hz, H-6), 3.47 (1H, *part A syst. AB, J=* 13.5 Hz, H-3), 2.95 (1H, s, OH), 2.69 (1H, *part B syst. AB, J=* 13.5 Hz, H-3'), 1.18-0.94 (28H, m, TIPDS), 0.88 (9H, s, C(CH₃)₃), 0.08 (3H, s, SiCH₃), 0.04 (3H, s, SiCH₃).
**¹³C-NMR** (75MHz, CDCl₃): δ 203.1 (C-5), 167.3, 164.7, 135.6, 133.8, 129.7, 128.9, 128.6, 128.2, 78.6, 77.6 (C-6), 75.0 (C-7), 74.6 (C-8), 72.5 (C-4), 68.5 (C-9), 66.6, 39.9 (C-3), 25.6, 18.0, 17.7, 17.4, 17.35, 17.3, 17.2, 17.1, 17.0, 16.9, 13.9, 13.15, 13.1, 13.05, - 4.7, -5.6.
**IR** (film): ν 3398, 2947, 2889, 2862, 1763, 1725, 1602, 1588, 1461, 1411, 1385, 1322, 1266, 1150, 1119, 1067, 998, 950, 889, 872, 840, 778 cm⁻¹.
***LRMS** (API-ES⁺):* m/z 1650 (2M+Na)⁺, 836 (M+Na)¹, 814 (M+H)⁺.
**E.A.** (C₄₁H₆₃NO₁₀Si₃): Found: C, 60.59; H, 7.86; N, 1.95. Calculated: C, 60.48; H, 7.80; N, 1.72.

### Example 2:

### Preparation of rac-(4S,6S,7R,8S,9S)-7-Benzoyloxymethyl-6-tert-butyldimethylsyliloxy-1,7,8,9-tetrahydroxy-8,9-O-(1,1,3,3-tetraisopropyldisyloxane-1,3-diyl)-1-azaspiro[3.5]nonane-2,5-dione (71)

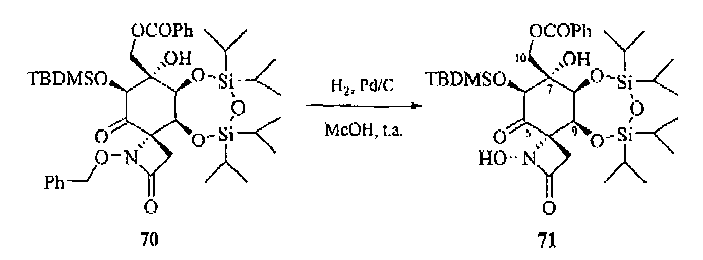

Pd/C (6mg, 10% w/w, 0,005 mmol, 0,06 eq.) was added to a solution of *rac-*(*4S,6S,7R,8S,9S*)-1-benzyloxy-7-benzoyloxymethyl-6-*tert*-butyldimethylsilyloxy-7,8,9-trihydroxy-8,9-*O*-(1,1,3,3-tetraisopropyldisiloxane-1,3-diyl)-1-azaspiro[3.5]nonane-2,5-dionc (**70**) 870 mg, 0,086 mmol, 1.0 eq.) in MeOH (2 ml) and the resultant suspension was stirred over 3 hours under hydrogen atmosphere. After that time, the mixture was filtered through Celite and the catalyst was washed with MeOH. After that, de solvent was eliminated under pressure to yield *rac*-(4S,6*S*,7*R*,8*S*,9*S*)-7-Benzoyloxymethyl-6-tert-butyldimethylsilyloxy-1,7,8,9-tetrahydroxy-8,9-O-(1,1,3,3-tetraisopropyldisiloxane-1,3-diyl)-1-azaspiro[3.5]nonane-2,5-dione (71) (40 mg., 64% yield) as a white solid.
***R_{f}** =* 0.22 (TLC, hexane/AcOEt, 3:1).
**¹H-NMR** (300MHz, CDCl₃): δ 8.06 (2H, dm, *J =* 7.1 Hz, Bz), 7.59 (111, tm, *J* = 7.3 Hz, Bz), 7.46 (211, dd, *J =* 7.3, 7.1 Hz, Bz), 4.87 (1H, d, *J* = 4.0 Hz, H-9), 4.76 (1H, *part A syst*. *AB*, *J =* 11.7 Hz, H-10), 4,59 (1 H, dd, *J =* 4.0, 1.0 Hz, H-8), 4.51 (1H, *part B syst*. *AB*, *J =* 11.7 Hz, H-10'), 4.14 (1H, d, *J =* 1.0 Hz, H-6), 3.60 (1H, s ancho, HO-N), 3.47 (1H, *part A syst*. *AB*, *J =* 13.5 Hz, H-3), 3.47 (1H, s, HO-C(7)), 2.69 (1H, *part B syst*. *AB*, *J =* 13.5 Hz, H-3'), 1.18-0.96 (28H, m, TIPDS), 0.85 (9H, s, C(CH₃)₃), 0.04 (3H, s, SiCH₃), -0.01 (3H, s, SiCH₃).
**¹³C-NMR** (75MHz, CDCl₃): δ 204.9, 167.2, 164.5, 133.7, 129.7, 129.0, 128.6, 77.4, 74.9, 74.3, 71.8, 68.3, 66.5, 39.7, 25.5, 18.0, 17.6, 17.5, 17.4, 17.2, 17.1, 17.0, 16.9, 13.9, 13.0, -4.8, -5.7.
***LRMS (API-ES⁺)***: m/z 746 (M+Na)⁺, 724 (M+H)⁺.

### Example 3:

### Preparation of rac-(4S,6S,7R,8S,9S)-7-Benzoyloxymethyl-6-tert-butyldimethylsilyloxy-7,8,9-trihydroxy-1-(N,N'-diisopropylcarbamimidoyloxy)-8,9-O-(1,1,3,3-tetraisopropyldisiloxane-1,3-diyl)-1-azaspiro[3.5]nonane-2,5-dione (72)

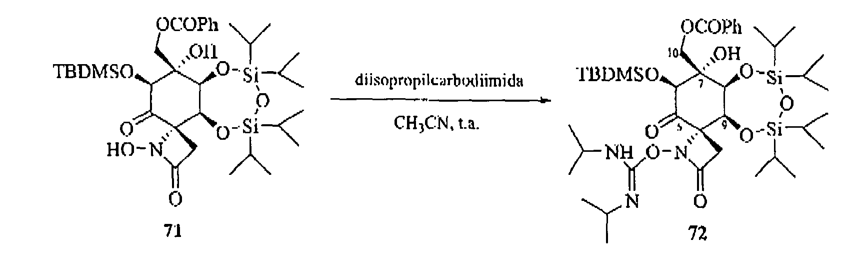

Diisopropylcarbodiimide (8 µl, 0,052 mmol, 1.0 eq.) was added at room temperature to a solution of *rac*-(4*S*,6*S*,7*R*,8*S*,9*S*)-7-Benzoyloxymethyl-6-*tert-*butyldimethylsiloxy-1,7,8,9-tetrahydroxy-1-8,9-*O*-(1,1,3,3-tetraisopropyldisiloxane-1,3-diyl)-1-azaspiro[3.5]nonane-2,5-dione (**71**) (38 mg, 0,052 mmol, 1.0 eq.) in CH3CN (1 ml). After 36 hours, more diisopropylcarbodiimide (1.0 eq.) was added and the resultant mixture was stirred over 5 hours at room temperature.
After that time, dc solvent was removed under reduced pressure and the residue was purified by column chromatography (hexane/AcOEt, 10:1) obtaining *rac-*(4*S*,6*S*,7*R*,8*S*,9*S*)-7-Benzoyloxymethyl-6-tert-butyldimethylsilyloxy-7,8,9-trihydroxy-1-(N,N'-diisopropylcarbamimidoyloxy)-8,9-O-(1,1,3,3-tetraisopropyldisiloxane-1,3-diyl)-1-azaspiro[3.5]non.ine-2,5-dione (72) (24 mg. 54% yield), as a white solid. Two by-products ((mg Rf- 0,59 (TLC, hexane/AcOEt, 3:1) and 7 mg Rf = 0.36 (TLC, hexane/AcOEt, 3:1) and the starting hydroxyamida 71 (2 mtg., 5%) where also isolated.
**P.f**.: °C.
***R_{f}*=** 0.69 (TLC, hexano/AcOEt, 3:1).
**¹H-RMN** (300MHz, CDCl₃): δ
**¹³C-RMN** (75MHz, CDCl₃): δ
**IR** (film): ν cm⁻¹,
***LRMS** (API-ES⁺):* m/z
**A.E.** (C₄₁H₇₁N₃O₁₀Si₃): Found: C,; H,; N,. Calculated: C, 57.92; H, 8.42; N, 4.94.

### Example 4:

### Preparation of rac-(4R, 5S, 6S, 7S, 8S, 9S)-1-Benzyloxi-7-benzoyloxymethyl-8,9-epoxi-5,6,7-trihydroxy-5,6-O-(1,1,3,3-tetraisopropyldisiloxane-1,3-diyl)-1-azaspiro[3.5] nonan-2-one (61)

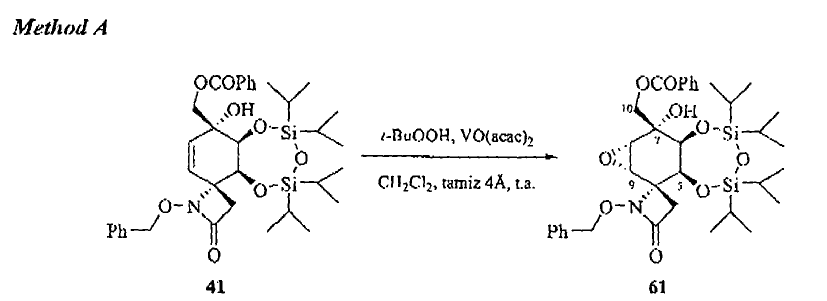

*Tert*-butyl hydroperoxide (86 µl, 80% weight solution in (*t*-BuO)₂O, 0.689 mmol, 10.0 eq.) and molecular sieves 4Å were added at room temperature to a solution of *rac-*(4*R, 5S, 6S,* 7*R*)-1-benzyloxi-7-benzoyloxymethyl-5,6,7-trihydroxy-5,6-*O-*(1,1,3,3,tetraisopropyldisiloxane-1,3-diyl)-1-azaspiro[3,5]non-8-en-2 one **(41)** (46 mg., 0.069 mmol, 1.0 eq.) in CH₂Cl₂ (0.6 ml). After 10 minutes, VO(acacc)2 (5mg, 0.021 mmol, 0,3 eq.) was added. The resultant mixture was stirred at room temperature over 4 days. After that time, de mixture was diluted with CH₂Cl₂ (3 ml) and was filtered under reduced pressure through Celite to eliminate the sieves. The solution was washed with an aqueous solution of NaHCO₃ 10% (2 ml), dried with anhydrous Na₂SO₄, filtered and the solvent was removed under reduced pressure. The product was purified by column chromatography (hexane/AcOEt, 4:1) and *rac-*(4*R,* 5*S*, 6*S*, 7*S*, 8S, 9*S*)-1-Benzyloxi-7-benzoyloxymethyl-8,9-epoxi-5,6,7-trihydroxy-5,6-*O*-(1,1,3,3-tetraisopropyldisiloxane-1,3-diyl)-1-azaspiro[3.5]nonan-2-one (**61**) (41 mg, 87% yield) was obtained as a white solid. 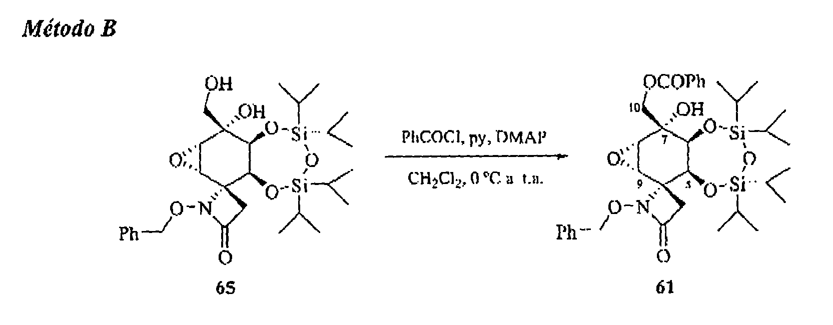 Pyridine (2 µl, 0.024 mmol, 2.0 eq.), a catalytic amount of 4-dimethylaminopyridine (0.01 eq.) and benzoyl, chloride (2 µl, 0.014 mmol, 1.4 eq.) were sequentially added at 0°C, to a solution of *rac-(4R, 5S, 6S, 7S,* 8*S*, 9*S*)-1-benzyloxi-8,9-epoxi-5,6,7-trihydroxy-7-hydroxymethyl-5,6-*O*-(1,1,3,3-tetraisopropyldisiloxane-1,3-diyl)-1-azaspiro[3.5]nonan-2-one (**65**) (87 mg, 0.012 mmol, 1.0 eq.) in CH₂Cl₂ (0.15 ml). After 18 hours at room temperature, more pyridine (2.0 eq.), DMAP (0.01 eq.) and benzoyl chloride (1.4 eq,) at 0°C were added, and the resulting mixture was stirred at room temperature over 18 hours. After that time, the medium was neutralized by addition of saturated NaHCO₃ and the mixture was extracted with Et₂O (3 x 2ml). The organic phase was washed with HCl 10% (2 ml) and NaCl saturated (2 ml), dried with anhydrous MgSO₄, filtered and the solvent eliminated under reduced pressure. The product was purified by column chromatography (hexane/ Ac=Et, 6:1) obtaining *rac-*(4*R*, *5S, 6S, 7S,* 8*S*, 9*S*)-1-Benzyloxi-7-benzoyloxymethyl-8,9-epoxi-5,6,7-trihydroxy-5,6-*O*-(1,1,3,3-tetraisopropyldisiloxane-1,3-diyl)-1-azaspiro[3.5]nonan-2-one (**61**) 85 mg, 62% yield) as a white solid.
*R_{f}=* 0.36 (TLC, hexane/AcOEt, 3:1); 0.51 (TI.C, hexanc/AcOEt, 2:1).
**¹H-NMR** (200MHz, CDCl₃): δ 8.07 (2H, dm, *J =* 7.0 Hz, Bz), 7.60 (1H, tm, *J* = 7.3 Hz, Bz), 7.53-7.41 (4H, m, Bz y Ph), 7.41-7.31 (3H, m, Ph), 5.12 (1H, *part. A .syst. AB, J=* 11.0 Hz, OCH₂Ph), 5.03 (1H, *part B syst. AB, J =* 11.0 Hz, OCH₂Ph), 4.58 (1H, *part A. syst. AB, J* = 12.0 Hz, H-10), 4.53 (1H, s broad, H-5), 4.52 (1H, *part B syst. AB, J* = 12.0 Hz, H-10'), 4.24 (1H, s broad, H-6), 3.52 (2H, s broad, H-8 y H-9), 3.16 (1H, *part A syst. AB, J=* 13.9 Hz, H-3), 2.81 (1 H, s, OH), 2.48 (1H, *part B syst. AB, J* = 13.9 Hz, H-3'), 1.13-0.98 (28H, m, TIPDS).
**¹H-NMR** (300MHz, C₆D₆): δ 8.16 (2H, dm, *J =* 8.3 Hz, Bz), 7.50 (1H, d, *J =* 7.6 Hz, Bz), 7.20-7.01 (7H, m, Bz y Ph), 5.10 (1H, *part A syst. AB, J=* 11.5 Hz, OCH₂Ph), 5.06 (1H, *part B syst. AB, J =* 11.5 Hz, OCH₂Ph), 4.76 (1H, t, *J* = 1.9 Hz, H-5 o H-6), 4.52 (1H, *part A syst. AB, J=* 11.7 Hz, H-10), 4.45 (1H, *part B syst. AB, J =* 11.7 Hz, H-10'), 4.28 (1H, m, H-6 o H-5), 3.17 (1H, d, *J* = 13.7 Hz, H-3), 3.02 (1H, m, H-8 o H-9), 2.96 (1H, dd, *J* = 3.7, 1.9 Hz, H-9 o II-8), 2.73 (1H, m, OH), 2.18 (1H, dd, *J*= 13.7, 1.9 Hz, H-3'), 1.35-0.80 (28H, m, TIPDS).
**¹H-RMN** (500MHz, CO(CD₃)₂): δ 8.12 (2H, m, Bz), 7.66 (1H, m, Bz), 7.57-7.46 (4H, m, Bz y Ph), 7.42-7.32 (3H, m, Ph), 5.12 (1H, *part A syst. AB*, *J=* 10.9 Hz, OCH₂Ph), 5.06 (1H, *part B syst. AB*, *J =* 10.9 Hz, OCH₂Ph), 4.98 (11-1, s, OH), 4.88 (1H, d, *J* - 2.4 Hz, H-5), 4.59 (1 H, *part A syst. AB*, *J =* 11.5 Hz, H-10), 4.54 (111, *part B syst*. *AB*, *J =* 11.5 Hz, H-10'), 4.37 (1H, dd, *J* = 2.4, 1.3 Hz, H-6), 3.71(1H, d, *J* = 3.7 Hz, H-9), 3.64 (1 H, dd, *J* = 3.7, 1.3 Hz,H-8), 3,08 (1H, *part A syst. AB*, *J =* 13.5 Hz, H-3), 2.68 (1H, *part B syst. AB, J* - 13.5 Hz, H-3'), 1.23-0.92 (28H, m, TIPDS).
¹³**C-RMN** (75MHz, CDCl₃): δ 166.4, 163.9. 135.2, 133.4, 129.6, 129.5, 128.9, 128.7, 128.5, 128.4, 78.8, 76.6 (C-6), 71.4 (C-7), 66.8 (C-10), 66.2 (C-5), 65.5 (C-4), 60.3 (C-9), 55.0 (C-8), 38.3 (C-3), 17.6,17.5, 17.2, 17.1, 17.0, 16.9, 14.3, 14.2, 13.5, 13.2.
¹³**C-RMN** (125MHz, CO(CD₃)₂): δ 166.9, 164.4, 136.9, 134.2, 131.2, 130.5, 129.8, 129.5, 129.4, 129.3, 79.2, 78.5 (C-6), 72.3 (C-7), 68.7 (C-10), 67.8 (C-5), 66.6 (C-4), 59.8 (C-9), 56.2 (C-8),38.8 (C-3), 18.2, 18.1, 17.7, 17.6, 17.55, 17.5, 15.4, 15.2, 14.4, 14.0.
**IR** (film): ν 3430, 2947, 2900, 2868, 1767, 1725, 1599, 1589, 1465, 1451, 1394, 1370, 1273, 1148, 1104, 1075, 1007, 941, 886, 823, 756, 711 cm⁻¹.
***LRMS*** (*API-ES⁺*): m/z 1389 (2M + Na)⁺, 706 (M+Na)⁺, 684 (M+H)¹.
**A.E**. (C₃₅H₄₉NO₉Si₂). Hallado: C, 61.54; H, 7.37; N, 2.21. Calculado: C, 61.46; H, 7.22; N, 2.05.

### Example 5:

### Preparation of rac-(4R,5S,6S,7S,8S,9S)-7-benzoyloximethyl-8,9-epoxi-1,5,6,7-tetrahydroxi-5,6-O-(1,1,3,3-tetraisopropyldiisosiloxano-1,3-diyl)-1-azaspiror[3.5]nonan-2-one (73)

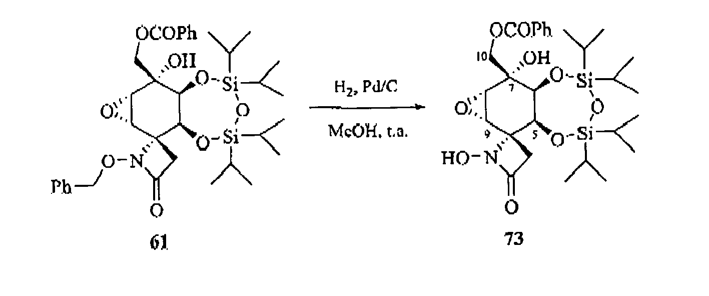

Over a solution of *rac-(*4*R,* 5*S*, 6*S*, 7*S,* 8*S*, 9*S*)-1-Benzyloxi-7-benzoyloxymethyl-8,9-epoxi-5,6,7-trihydroxy-5,6-*O*-(1,1,3,3-tetraisopropyldisiloxane-1,3-diyl)-1-azaspiro[3.5]nonan-2-one (61) (90 mg, 0.132 mmol, 1.0 eq.) in MeOH (1.6 ml) Pd/C was added (8 mg, 10% wt, 0.008 mmol, 0.06 eq.) and the resulting suspension was stirred under hydrogen for 3 hours, after which the mixture was filtrated through Celite and the catalyst washed with MeOH. Removal of the solvent yielded *rac-*(4*R*,5*S*,6*S*,7*S*,*8*S,9*S*)-7-benzoyloximethyl-8,9-epoxi-1,5,6,7-tetrahydroxi-5,6-*O*-(1,1,3,3-tetraisopropyldiisosiloxane-1,3-diyl)-1-azaspiro[3.5]nonan-2-one (73) (78 mg, quantitative yield) as a white solid.
***R_{f}*** = 0.08 (TLC, hexane/AcOEt, 2:1); 0.48 (TLC, hexane/AcOEt, 1:3); 0.20 (TLC, hexane/AcOEt, 1:1).
**¹H-NMR** (200MHz, CDCl₃): δ 8.07 (2H, d, *J* = 7.6 Hz, Bz), 7.60 (1H, t, *J =* 7.3 Hz, Bz), 7.46 (2H, dd, *J* = 7.6, 7.3 Hz, Bz), 4.67 (1H, d, *J* = 1.7 Hz, H-5), 4.60 (1H, *part A syst*. *AB*, *J =* 12.1 Hz, H-10), 4.53 (1H, *part B syst*. *AB*, *J* = 12.1 Hz, H-10'), 4.28 (1H, s broad, H-6), 3.60 (1H, d, *J* = 3.6 Hz, H-9), 3.49 (2H, s broad, H-8 y HO-N), 3.21 (1H, *part A syst*. *AB*, *J* = 13.8 Hz, H-3), 2.87 (1H, s broad, HO-C(7)), 2.54 (1H, *part B syst*. *AB*, *J =* 13.8 Hz, H-3'), 1.15-0.95 (28H, m, TIPDS).
**¹H-NMR** (500MHz, CO(CD₃)₂): δ 9.10 (1H, s broad, HO-N), 8.11 (2H, dm, *J* = 7.1 Hz, Bz), 7.66 (1H, tm, *J* = 7.5 Hz, Bz), 7.53 (2H, ddm, *J* = 7.5, 7.1 Hz, Bz), 4.83 (1H, s, HO-C(7)), 4.80 (1H, d, *J* = 2.4 Hz, 11-5), 4.57 (1H, *part A syst*. *AB*, *J* = 11.5 Hz, H-1 0), 4-53 (1H, *part B syst. AB*, *J =* 11.5 Hz, H-10'), 4.35 (1H, dd, *J* = 2.4, 1.5 Hz, H-6), 3.61 (1H, d, *J* = 3.7 Hz, H-9), 3.49 (1H, dd, *J* = 3.7, 1.5 Hz, H-8), 3.04 (1H, *part A syst. AB*, *J =* 13.3 Hz, H-3), 2.61 (1H, *part B syst. AB, J =* 13.3 Hz, H-3'), 1.20-0-95 (2811, m, TIPDS).
**¹³C-NMR** (125MHz, CO(CD₃)₂): δ 166.9, 164.4, 134.1, 131.2, 130.5, 129.4, 78.5 (C-6), 72.4 (C-7), 68.7 (C-10), 67.5 (C-5), 65.7 (C-4), 59.8 (C-9), 54.4 (C-8), 38.8 (C-3), 18.2, 18.1, 18.05, 17.7, 17.6, 17.5, 17.45, 15.3, 15.25, 14.4, 14.0.
**IR** (KBr): ν 3436, 3268 (shoulder), 2947, 2889, 2862, 1748, 1729, 1631, 1465, 1455, 1411, 1389, 1372, 1273, 1147, 1104, 1072, 1006, 942, 885, 825 cm⁻¹.
***LRMS** (API-ES¹):* m/z. 1232 (2M+2Na)⁺, 1209 (2M+Na)⁺, 616 (M+Na)⁺, 594 (M+H)⁺.
***LRMS** (EI):* m/z 593 (M⁺, 2), 561 (3), 550 (3), 534 (1), 459 (1), 440 (1), 428 (3), 413 (2), 384 (2), 365 (3), 339 (17), 289 (7), 261 (7), 235 (5), 175 (5), 163 (5), 147 (7), 135 (12), 119 (8), 105 (100), 77 (15).
**E.**A. (C₂₈H₄₃NO₉Si₂): Found: C, 56.75; H, 7.46; N, 2.57. Calculated: C, 56.63; H, 7.30; N, 2.36.

### Example 6:

### Preparation of rac-(4R,5S,6S,7S,8S,9S)-7-Benzoyloxymethyl-8,9-epoxy-5,6,7-trihydroxy-1-(N,N'-diisopropylcarbamidoylnxy)-5,6-O-(1,1,3,3-tetraisopropyldisyloxane-1,3-diil)-1-azaspiro[3.5]nonan-2-one (74)

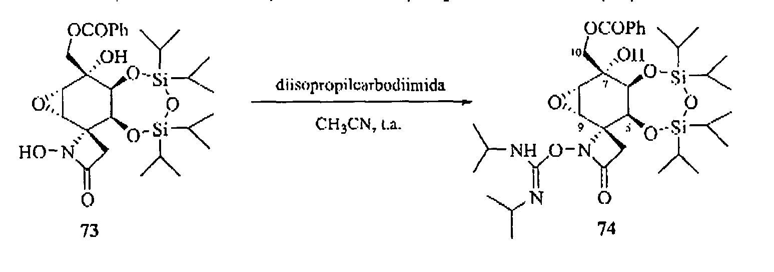

Diisopropylcarbodiimide (10 µl, 0.057 mmol, 1.0 eq.) was added to a stirred solution of *rac-*(4*R*,5*S*,6*S*,7*S*,8*S*,9*S*)-7-benzoyloxymethyl-8,9-epoxy-1,5,6,7-tetrahydroxy-5,6-*O*-(1,1,3,3-tetraisopropyldisyloxane-1,3-diil)-1-azaspiro[3.5]nonan-2-one (73) (34 mg, 0.057 mmol, 1.0 eq.) in CH₃CN (1 ml). After 36 hours, more diisopropylcarbodiimide (0.5 eq.) was added and the resulting suspension stirred at room temperature for 3 days, after which, the solvent was removed under reduced pressure to obtain *rac-*(4*R*,5*S*,6*S*,7*S*,8*S*,9*S)-*7-Benzoyloxymethyl-8,9-epoxy-5,6,7-trihydroxy-1-(*N,N'*-diisopropylcarbamimidoyloxy)-5,6-*O*-(1,1,3,3-tetraisopropyldisyloxane-1,3-diil)-1-azaspiro[3.5]nonan-2-one (74) (30 mg, yield 73%) as a white solid.
***R_{f}**-* 0.62 (TLC, hexane/AcOEt, 2:1); 0.79 (TLC, hexane/AcOEt, 1:1).
**¹H-NMR** (300MHz, CDCl₃): δ 8.08 (2H, dm, *J* = 7.2 Hz, Bz), 7.58 (1H, tm, *J* = 7-4 Hz, Bz), 7.45 (2H, ddm, *J* = 7.4, 7.2 Hz, Bz), 6.16 (1H, s broad, NH), 4.91 (1H, *part A syst. AB, J*= 12.1 Hz, H-10), 4.76 (1H, h, *J* = 6.8 Hz, NCH(CH₃)₂), 4.56 (1H, *part B syst. AB, J* - 12.1 Hz, H-10'), 4.41 (111, s broad, H-5), 4.32 (1H, d, *J* = 2.5 Hz, H-6), 3.87 (1H, h, *J* = 6.3 Hz, NCH(CH₃)₂), 3.68 (1H, d, *J* = 3.7 Hz, H-8), 3.56 (1H, dd, *J* = 3.7, 1.0 Hz, H-9), 2.86 (1H*, part A syst. AB* broad, *J* = 13.4 Hz, H-3), 2.84 (1H, s broad, IIO-C(7)), 2.69 (1H, *part B syst. AB, J* = 13.4 Hz, H-3'), 1.38 (3H, d, *J* = 6.8 Hz, NCH(CH₃)₂), 1.25 (3H, d, *J* = 6.8 Hz, NCH(CH₃)₂), 1.18-0.96 (34H, m, TIPDS y NCH(CH₃)₂).
**¹³C-NMR** (75MHz, CDCl₃): δ 169.0, 166.9, 146.4, 133.3, 129.7, 128.5, 74.4, 73.2, 66.2 63.1, 56.6, 55.8, 48.6, 46.4, 42.2, 40.8, 23.7, 23.5, 21.4, 19.4, 17.8, 17.6, 17.55, 17.5, 17.2, 17.15, 17.1, 16.8, 14.2, 13.8, 13.6, 13.3.
***LRMS*** (*API-ES⁺*): m/z 1462 (2M+Na)⁺, 720 (M + H)⁺,
***LRMS** (EI):* m/z 719 (M⁺, 0.2), 676 (2), 658 (1), 634 (2), 619 (3), 591 (2), 561 (3), 550 (2), 534 (2), 419 (3), 413 (2), 395 (2), 365 (4), 339 (24), 289 (6), 261 (11), 235 (4), 163 (5), 147 (6), 127 (18), 105 (100), 77 (16).

### Example 7:

### Preparation of rac-(4S,5R,6R,7R,8S,9S)-1-Bencyloxy-7-benzoyloxymethyl-6-tert-butyldimethylsyliloxy-5-cyano-8,9-dihydroxy-8,9-O-(1,1,3,3-tetraisopropyldisiloxane-1,3-diil)-5,7-bis(methoxycarbonoyloxy)-1-azaspiro[3.5]nonan-2-one (75)

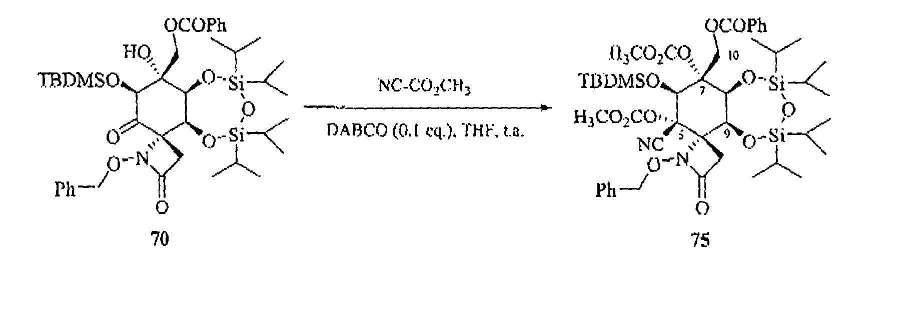

To a solution of *rac-*(4*S,*6*S,*7*R,*8*S,*9*S)-*1-bencyloxy-7-benzoyloxymethyl-6-*tert*-butyldimethylsyliloxy-7,8,9-trihydroxy-8,9-*O*-(1,1 ,3,3-tetraisopropyldisyloxane-1,3-diil)-1-azaspiro[3.5]nonane-2,5-dione (70) (85 mg, 0,104 mmol, 1.0 eq.) and methyl cyanoformiate (100 µl, 1.248 mmol, 12.0 eq.) in THF (1 ml) DABCO was added at room temperature (3 mg, 0.026 mmol, 0.25 eq.). The resulting mixture was stirred at room temperature for 15 hours, after which solvent was removed under reduced pressure. The residue was grinded with AeOEt, filtered through Celite and the solvent removed under reduced pressure. The product was purified by silica gel column chromatography (hexane/AcOEt, 8:1) and *rac*-(4*S*,5*R*,6*R*,7*R*,8*S*,9*S*)-1-Bencyloxy-7-benzoyloxymethyl-6-*tert*-butyldimethylsyliloxy-5 -cyano-8,9-dihydroxy-8,9-*O-*(1,1,3,3-tetraisopropyldisiloxane-1,3-diil)-5,7-bis(methoxycarbonoyloxy)-1-azaspiro[3.5]nonan-2-one (75) (29 mg, yield. 29%) was obtained as a white solid. An isomer was also isolated (5 mg, rto. 5%, *R_{f} =* 0.49 (TLC, hexane/AcOEt, 3:1)).
***R_{f}** =* 0.41 (TLC, hexane/AcOEt, 3:1).
**¹H-NMR** (300MHz, CDCl₃): δ 7.98 (2H, , *J* = 6.9 Hz, Bz), 7.61 (1H, *J* = 7.4 Hz, Bz), 7.53-7.41 (4H, m, Bz y Ph), 7.41-7.28 (3H, m, Ph), 5.56 (1H, d, *J* = 1.3 Hz, H-6), 5.14 (1H, dd, *J* = 4.0, 1.3 Hz, H-8), 5.08 (1H, *part A* syst. *AB, J=* 12.0 Hz, H-10), 5.05 (1H, *pare A syst. AB, J* - 10.1 Hz, OCH̅₂Ph), 5.00 (1H, *part* B *syst. AB*, *J =* 10.1 Hz, OCH₂Ph), 4.88 (1H, d, *J* = 4.0 Hz, H-9), 4.83 (1H, *part B syst. AB*, *J =* 12.0 Hz, H-10'), 3.90 (3H, s, OCH₃), 3.62 (3H, s, OCH₃), 3.45 (1H, *part A syst. AB*, *J =* 14.8 Hz, H-3), 3.05 (111, *part B syst. AB*, *J* = 14.8 Hz, H-3'), 1.16-0.88 (28H, m, TIPDS), 0.95 (9H, s, C(CH₃)₃), 0.40 (3H, s, SiCH₃), 0.23 (3H, s, SiCH₃).
**¹³C-NMR** (75MHz, CDCl₃): δ 167.0, 165.4, 152.9, 152.7, 135.3, 133.5, 129.4, 129.2, 128.9, 128.6, 128.3, 128.2, 115.1 (CN), 83.6 (C-7), 81.9 (C-5), 78.7, 71.0 (C-8), 68.7 (C-6), 66.5 (C-4), 65.2 (C-9), 61.9 (C-10), 55.8, 55.1, 38.8 (C-3), 26.4, 18.6, 17.6, 17.4, 17.3, 17.1, 17.0, 16.9, 16.8, 16.7, 13.5, 13.3, 13.1, 13.0, -3.3, -4.0.
**IR** (KBr): ν 3436, 2946, 2889, 2862, 2338, 1794, 1776, 1751, 1729, 1633, 1465, 1440, 1394, 1378, 1293, 1263, 1158, 1121, 1089, 1044, 995, 977, 922, 840, 785, 712 cm⁻¹.
***LRMS** (API-ES⁺):* m/z 1936 (2M+Na)⁺, 1915 (2M+H)¹, 1029 (M+73)⁺, 957 (M+H)⁺.

### Example 8:

### Preparation of rac-(4R,5S,6S,9R)-1-benzyloxy-6-tert-butyldimethylsilyloxy-9-cyano-5-trimethylsilyloxy-1-azaspiro[3.5]nona-8-en-2,7-dione

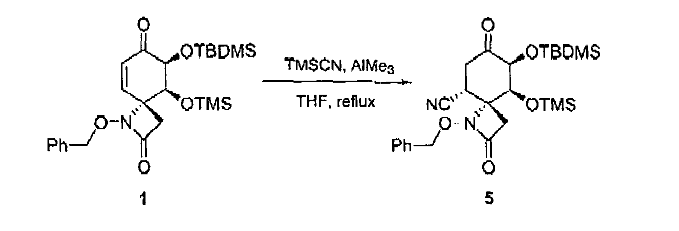

To a stirred solution of cyanotrimethyl silane (56 µl, 0.411 mmol) and trimethylaluminium (0.19 ml, 2.0 M solution in toluene, 0.374 mmol) in THF (0.5 ml) was added at room temperature a solution of *rac*-(4*R*,5*S*,6*S*)-1-benzyloxy-6-*tert-*butyldimethysilyloxy-5-trimethylsilyloxy-1-azaspiro[3.5]nona-8-en-2,7-dione (1) (89 mg, 0.187 mmol) in THF (0.5 ml). The resulting mixture was stirred at reflux for 24 h and then concentrated under reduced pressure. The residue was dissolved in toluene (2 ml), was washed with cold NH₄Cl sat. (1 ml) and Na₂HPO₄ 0.1 M buffer (1 ml), dried over Na₂SO₄, filtered and concentrated under reduced pressure. The residue was purified by silica gel column chromatography (hexane-AcOEt, 5:1) to give *rac*-(4*R*,5*S*,6*S*,9*R*)-1-benzyloxy-6-*tert-*butyldimethylsilyloxy-9-cyano-5-trimethylsilyloxy-1-azaspiro[3.5] nona-8-en-2,7-dione (5) as a low melting point white solid (21 mg, 22%).
***R_{f}** =* 0.41 (TLC, hexane-AcOEt, 3:1); yield, 22%; white solid.
**¹H-NMR** (300MHz, CDCl₃): δ 7.44-7.30 (5H, m, Ph), 5.31 (1H, *part A* syst. *AB, J =* 10.7 Hz, OCH₂Ph), 5.11 (1H, *part B syst. AB,* J = 10.7 Hz, OCH₂Ph), 4.71 (1H, d, J = 2.2 Hz, H-6), 4.13 (1H, d, J - 2.2 Hz, H-5), 3.66 (1H, dd, J - 13.4, 5.4 Hz, H-9), 2.92 (1H, *part A syst. AB*, J *=* 14.5 Hz, H-3), 2.76 (1H, *part B syst. AB*, J - 14.5 Hz, H-3), 2.63 (1H, dd, J = 13.4, 5.4 Hz, H-8), 2.21 (1H, t, J = 13.4 Hz, H-8), 0.85 (9H, s, C(CH₃)₃), 0.09 (9H, s, Si(CH₃)₃), 0.08 (3H, s, SiCH₃), -0.06 (3H, s, SiCH₃),
**¹³C-NMR** (75MHz, CDCl₃): δ 201.0, 164.1, 1.33.9, 129.5, 129.4, 128.9, 117.1, 80.8, 78-9, 78.0, 67.3 , 42.2, 38.3, 32.0, 25.8, 1 8,5, 0.5, -4.8, -5.6.
**IR (KBr)**: ν 3425, 2956, 2927, 2855, 2233, 1772, 1631 , 1454, 1364, 1255, 1107, 1064, 841 cm⁻¹.
**LRMS**(API-ES⁺): m/z 525(M+Na)⁺, 503(M+H)⁺.

### Example 9:

### Preparation of rac-(4R,5S,6S,9R)-9-Cyano-1,5,6-trihydroxy-5,6-O-(1,1,3,3-tetraisopropyidisiloxane-1,3-diil)-1-azaspiro[3.5]nonane-2,7-dione (77)

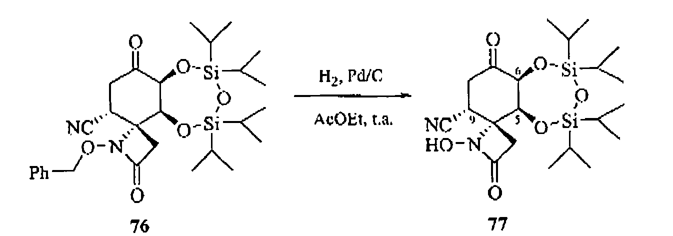

To a solution of *rac*-(4*R*,5*S*,6*S*,9*R*)-1-bencyloxy-9-ciano-5,6-dihydroxy-5,6-*O*-(1,1,3,3-tetraisopropyldisyloxane-1,3-dill)-1-azaspiro[3.5]nonanr-2,7-dione (76) (110 mg, 0.197 mmol, 1.0 eq.) in AcOEt (2 ml) Pd/C was added (13 mg, 10% w/w, 0.012 mmol, 0.06 eq.) and the resulting suspension was stirred under hydrogen atmosphere for 1 hour. Afterwards the mixture was filtered with celite and the catalyst was first washed with MeOH and then with AcOEt. Next, the solvent was removed from the alcohol phase under reduced pressure and the residue purified by column chromatography (hexane/AcOEt, 5:1). *rac*-(4*R*,5*S*,6*S*,9*R*)-9-Cyano-1,5,6-trihydroxy-5,6-*O*-(1,1,3,3-tetraisopropyldisiloxane-1,3-diil)-1-azaspiro[3.5]nonane-2,7-dione (77) (27 mg, yield 29%) was obtained as a white solid. The AcOEt extract was also evaporated under reduced pressure and starting material (7 mg, 6%, *R_{f} =* 0.62 (TLC, hexane/AcOEt, 3:1)) was recovered.
***R_{f} =*** 0.20 (TLC, hexano/AcOEt, 3:1).
**¹H-NMR** (300MHz, CDCl₃): δ 10.5 (1H, s broad, OH), 5.25 (1 H, d, *J* = 2.4 Hz, H-5 o H-6), 4.54 (1H, d, *J*= 2.4 Hz, H-6 o H-5), 3.76 (1H, dd, *J*= 12.7, 5.5 Hz, H-8), 3.11 (1H, t, *J* = 13.8 Hz, H-9), 2.94 (1H, *part A syst. AB, J* = 14.2 Hz, H-3), 2-87 (1H, *part B syst. AB, J* = 14.2 Hz, H-3'), 2.79 (1H, dd, *J* = 14.4, 5.5 Hz, H-8'), 1. 16-0.88 (28H, m, TIPDS).
***LRMS** (API-ES⁺):* m/z 960 (2M+Na)⁺, 491 (M+Na)¹, 469 (M+H)⁺.
***LRMS*** (*EI*): m/z 468 (M⁺, 9), 451 (4), 441 (1), 425 (100), 409 (12), 397 (13), 381 (19), 364 (33), 353 (6), 339 (39), 320 (36), 304 (13), 289 (10), 275 (43), 260 (15), 235 (23), 207 (12), 189 (18), 175 (17),163 (19), 147 (22), 135 (42), 119 (27), 105 (30), 91 (60), 77 (28).

## Claims

1. A process for the synthesis of a compound of formula I: 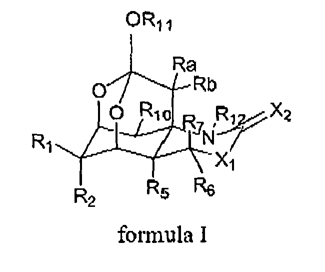 wherein
R₁ and R₂ together are =O or alkenyl; or
R₂ is selected from H, OH or OPr, and R₁ is selected from hydrogen, cyano, substituted or unsubstituted alkyl, alkyl-OPr, substituted or unsubstituted cycloalkyl, substituted or unsubstituted alkenyl, substituted or unsubstituted alkinyl, substituted or unsubstituted aryl, substituted or unsubstituted heterocyclyl;
R₅ is selected from H, OH, OPr;
R₆ and R₇ together are =O or =NPrn; or R₆ and R₇ are each independently selected from H, OH, OPr or cyano;
R₁₀ is selected from H, OH, OPr or -O;
R₁₁ is H, Pr or a negative charge;
R₁₂ is H or Prn;
X₁ is selected from NPrn, NH, O or S;
X₂ is selected from NPrn, NH, ⊕NH₂, O or S;
Ra and Rb are each independently selected from H, OH, OPr or substituted or unsubstituted alkyl, substituted or unsubstituted amino or halogen;
Pr is a hydroxyl protecting group;
Prn is a amino protecting group;
or its salts or tautomers,
comprising the step of forming a orthoester from a compound of formula VII: 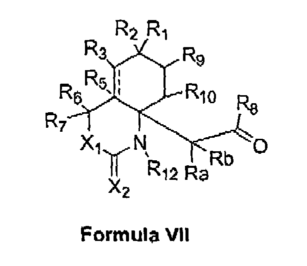 wherein
R₁ and R₂ together are =O or alkenyl; or
R₂ is selected from H, OH or OPr, and R₁ is selected from hydrogen, cyano, substituted or unsubstituted alkyl, alkyl-OPr, substituted or unsubstituted cycloalkyl, substituted or unsubstituted alkenyl, substituted or unsubstituted alkinyl, substituted or unsubstituted aryl, substituted or unsubstituted heterocyclyl;
R₆, and R₇ together are =O or =NPrn; or R₆ and R₇ are each independently selected from H, OH, OPr or cyano;
R₈ is H, OH, ester or amide;
R₉ and R₁₀ are each independently selected from H, OH, OPr or =O;
R₃ is selected from H, OH, OPr or -O,
if the bond between C5 and C4a is a single bond, R₅ is selected from H, OH or OPr; or R₃ and R₅ together are -O- forming an epoxide ring;
R₁₂ is H or Prn;
Ra and Rb are each independently selected from H, OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino or halogen;
Pr is a hydroxyl protecting group;
Prn is a amino protecting group;
X₁ is selected from NPrn, NH, O or S;
X₂ is selected from NPm, NH, ⊕NH₂, O or S.

2. A process for the synthesis of a compound of formula VII 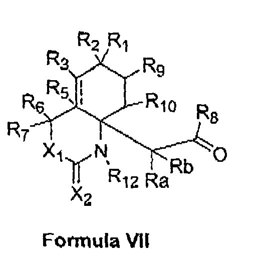 wherein
R₁ and R₂ together are =O or alkenyl; or
R₂ is selected from H, OH or OPr, and R₁ is selected from hydrogen, cyano, substituted or unsubstituted alkyl, alkyl-OPr, substituted or unsubstituted cycloalkyl, substituted or unsubstituted alkenyl, substituted or unsubstituted alkinyl, substituted or unsubstituted aryl, substituted or unsubstituted heterocyclyl;
R₆ and R₇ together are =O or =NPrn; or R₆ and R₇ are each independently selected from H, OH, OPr or cyano;
R₈ is H, OH, ester or amide;
R₉ and R₁₀ are each independently selected from H, OH, OPr or -O;
R₃ is selected from H, OH, OPr or =O,
if the bond between C5 and C4a is a single bond, R₅ is selected from H, OH or OPr; or R₃ and R₅ together arc -O-, forming an epoxide ring;
R₁₂ is H or Prn;
Ra and Rb are each independently selected from H, OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino or halogen;
Pr is a hydroxyl protecting group;
Prn is a amino protecting group;
X₁ is selected from NPm, NH, O or S;
X₂ is selected from NPrn, NH, ⊕NH₂, O or S;
which comprises opening the 1-aza-2-oxaspiro ring of a compound of formula VI 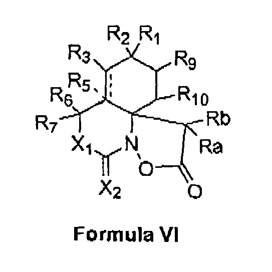 wherein X₁, X₂, R₁, R₂, R₃, R₅, R₆, R₇ R₉, R₁₀, Ra and Rb have the same meaning as in formula VII.

3. A process for the synthesis of a compound of formula 1: 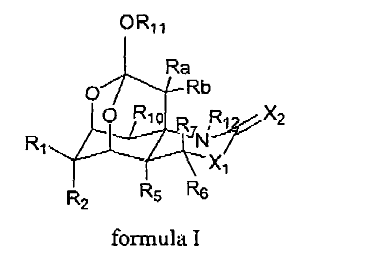 wherein
R₁ and R₂ together are -O or alkenyl; or
R₂ is selected from H, OH or OPr, and R₁ is selected from hydrogen, cyano, substituted or unsubstituted alkyl, alkyl-OPr, substituted or unsubstituted cycloalkyl, substituted or unsubstituted alkenyl, substituted or unsubstituted alkinyl, substituted or unsubstituted aryl, substituted or unsubstituted heterocyclyl;
R₅ is selected from H, OH, OPr;
R₆ and R₇ together are =O or =NPrn; or R₆ and R₇ are each independently selected from H, OH, OPr or cyano;
R₁₀ is selected from H, OH, OPr or =O;
R₁₁ is H, Pr or a negative charge;
R₁₂ is H or Prn;
X₁ is selected from NPrn, NII, O or S;
X₂ is selected from NPrn, NH, ⊕NH₂, O or S;
Ra and Rb are each independently selected from H, OH, OPr or substituted or unsubstituted alkyl, substituted or unsubstituted amino or halogen;
Pr is a hydroxyl protecting group;
Prn is a amino protecting group;
or its salts or tautomers,
comprising the following steps:
a) deprotecting the N-oxide residue of a compound of formula II 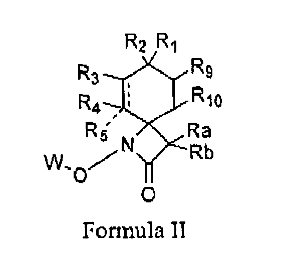 wherein
R₃ and R₉ are each independently selected from H, OH, OPr or =O;
R₄ is selected from cyano, substituted or unsubstituted alkyl, OPr, substituted or unsubstituted alkenyl, substituted or unsubstituted alkinyl;
if the bond between C5 and C4a is a single bond, R₅ is selected from H, OH or OPr; or R₄ and R₅ together are -O;
or R₃ and R₅ or R₃ and R₂ together are -O-, forming and epoxide ring;
W is a group comprising at least a group selected from substituted alkyl, substituted or unsubstituted aryl, substituted or usubstituted heterocyclyl, substituted or unsubstituted alkenyl;
R₁, R₂, R₁₀, Ra, Rb and Pr have the same meaning as in formula 1;
followed by the introduction of a residue of formula III 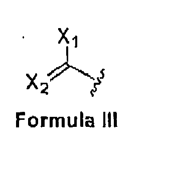 wherein
X₁ is selected from NHPrn, NH₂, N(Prn)₂, wherein Pm can be the same or different, OH, OPr, SH or SPrs;
X₂ is selected from NPrn, NH, O or S;
Prs is a sulfur protecting group;
Pr and Prn have the same meaning as in formula 1;
to give a compound of formula IV 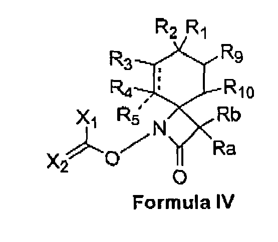 wherein
R₁, R₂, R₃, R₄, R₅, R₉, R₁₀, Ra and Rb have the same meaning as in formula II;
X₁ and X₂ have the same meaning as in formula III;
b) closing the ring between X₁ and either R₄ or C4a of the compound of formula IV to give a compound of formula V 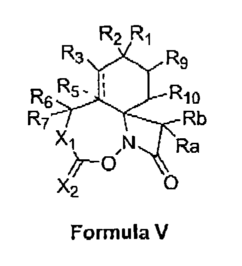 wherein
X₁ is selected from NPrn, NH, O or S;
X₂ is selected from NPrn, NH, ⊕NH₂, O or S;
R₁, R₂, R₃, R₅, R₉, R₁₀, Ra and Rb have the same meaning as in formula II;
R₆ and R₇ have the same meaning as in formula 1;
c) if necessary, subjecting the compound of formula V to a rearrangement to give a compound of formula VI 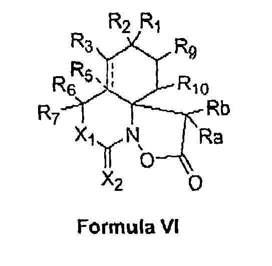 wherein
R₁, R₂, R₃, R₅, R₆, R₇, R₉, R₁₀, X₁, X₂, Ra and Rb have the same meaning as in formula V;
d) opening the 1-aza-2-oxaspiro residue of the compound of formula VI to give a compound of formula VII 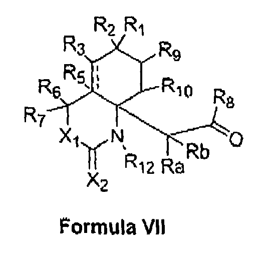 wherein
R₁ and R₂ together are =O or alkenyl; or
R₂ is selected from H, OH or OPr, and R₁ is selected from hydrogen, cyano, substituted or unsubstituted alkyl, alkyl-OPr, substituted or unsubstituted cycloalkyl, substituted or unsubstituted alkenyl, substituted or unsubstituted alkinyl, substituted or unsubstituted aryl, substituted or unsubstituted heterocyclyl;
R₆ and R₇ together are =O or =NPrn; or R₆ and R₇ are each independently selected from H, OH, OPr or cyano;
R₈ is H, OH, ester or amide;
R₉ and R₁₀ are each independently selected from H, OH, OPr or =O;
R₃ is selected from H, OH, OPr or =O,
if the bond between C5 and C4a is a single bond, R₅ is selected from H, OH or OPr; or R₃ and R₅ together are -O-, forming an epoxide ring;
R₁₂ is H or Prn;
Ra and Rb are each independently selected from H, OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino or halogen;
Pr is a hydroxyl protecting group;
Prn is a amino protecting group;
X₁ is selected from NPm, NH, O or S;
X₂ is selected from NPm, NH, ⊕NH₂, O or S; and
e) formation of the orthoester to give a compound of formula I from a compound of formula VII.

4. A process according to anyone of claims 1-3 wherein R₆ is OH or OPr and R₇ is H, or R₆ and R₇ together are -O.

5. A process according to anyone of claims 1-3 wherein R₁₀ is OH or OPr.

6. A process according to anyone of claims 1-3 wherein X₁ is N.

7. A process according to to anyone of claims 1-3 wherein R₅ is H.

8. A process according to claims 1 or 3 wherein the compound of formula I is TTX, its salts or tautomers thereof.

9. A process according to claims 1 or 3 wherein the step e) or formation of the orthoester is carried out by acid treatment.

10. A process according to claim 9 wherein the formation of the compound of formula I is carried out in two steps wherein
d₁) the compound of formula VII is treated with acid to give a compound of formula VIIIa 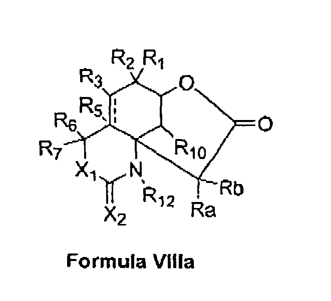 wherein
R₁, R₂, R₃, R₅, R₆, R₇, R₁₀, R₁₂, X₁, X₂, Ra, Rb, Pr and Prn have the same meaning as in formula VII, if R₉ is OH or OPr; or
d₂) the compound of formula VII is treated with acid to give a compound of formula VIIIb 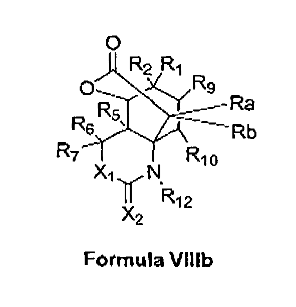 wherein
R₁, R₂, R₅, R₆, R₇, R₉, R₁₀, R₁₂ X₁, X₂, Ra, Rb, Pr and Prn have the same meaning as in formula VII if R₃ is OH or Pr.

11. Use of a compound of formula II 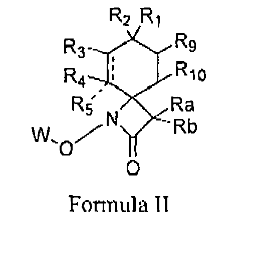 wherein
R₁ and R₂ together are =O or alkenyl; or
R₂ is selected from H, OH or OPr, and R₁ is selected from hydrogen, cyano, substituted or unsubstituted alkyl, alkyl-OPr, substituted or unsubstituted cycloalkyl, substituted or unsubstituted alkenyl, substituted or unsubstituted alkinyl, substituted or unsubstituted aryl, substituted or unsubstituted heterocyclyl;
R₉ and R₁₀ are each independently selected from H, OH, OPr or =O;
R₃ is selected from H, OH, OPr or =O;
R₄ is selected from cyano, substituted or unsubstituted alkyl, OPr, substituted or unsubstituted alkenyl, substituted or unsubstituted alkinyl;
if the bond between C5 and C4a is a single bond, R₅ is selected from H, OH or OPr; or
R₄ and R₅ together are =O; or
R₃ and R₅ or R₃ and R₂ together are -O-, forming an epoxide ring;
W is a group comprising at least a group selected from substituted alkyl, substituted or unsubstituted aryl, substituted or usubstituted heterocyclyl, substituted or unsubstituted alkenyl;
Ra and Rb are each independently selected from H, OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino or halogen;
Pr is a hydroxyl protecting group;
in the synthesis of tetrodotoxin, its tautomers, enantiomers or analogues.

12. Use according to claim 11 wherein the compound of formula 11 is selected from the group consisting of
compounds of formula IIa 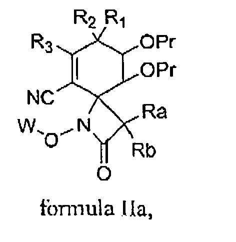
compounds of formula IIb 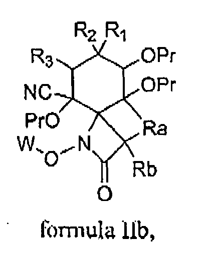
compounds of formula IIc 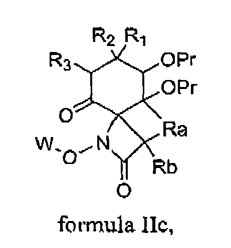
compounds of formula IId 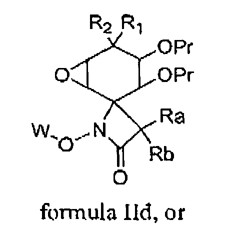
compounds of formula IIc 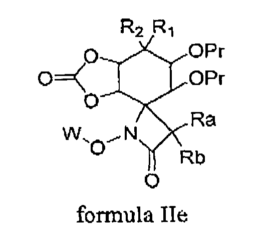 wherein R₁, R₂, R₃, Ra, Rb, W and OPr arc as defined in formula II.

13. A compound of formula VII 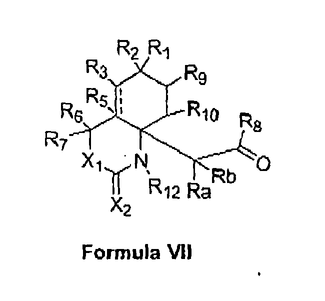 wherein
R₁ and R₂ together are =O or alkenyl; or
R₂ is selected from H, OH or OPr, and R₁ is selected from hydrogen, cyano, substituted or unsubstituted alkyl, alkyl-OPr, substituted or unsubstituted cycloalkyl, substituted or unsubstituted alkenyl, substituted or unsubstituted alkinyl, substituted or unsubstituted aryl, substituted or unsubstituted heterocyclyl;
R₆ and R₇ together are =O or =NPrn; or R₆ and R₇ are each independently selected from H, OH, OPr or cyano;
R₈ is H, OH, ester or amide;
R₉ and R₁₀ are each independently selected from H, OH, OPr or =O;
R₃ is selected from H, OH, OPr or =O,
if the bond between C5 and C4a is a single bond, R₅ is selected from H, OH or OPr; or R₃ and R₅ together are -O-, forming an epoxide ring;
R₁₂ is H or Prn;
Ra and Rb are each independently selected from H, OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino or halogen;
Pr is a hydroxyl protecting group;
Prn is a amino protecting group;
X₁ is selected from NPrn, NH, O or S;
X₂ is selected from NPrn, NH, ⊕NH₂, O or S.

14. A compound according to claim 13, of formula VIIa 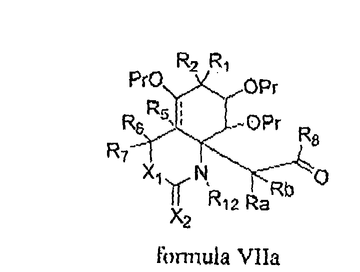 wherein R₁, R₂ R₅, R₆, R₇, R₈, R₁₂ Ra, Rb, X₁, X₂ and Pr are as defined claim 13.

15. A compound according to claim 13, of formula VIIb 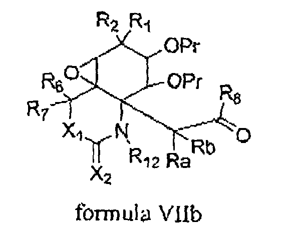 wherein R₁, R₂ R₆, R₇, R₈, R₁₂ Ra, Rb, X₁, X₂ and Pr are as defined claim 13.

16. A compound of formula VI 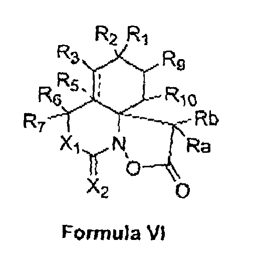 wherein R₁ ,R₂, R₃, R₅, R₆, R₇, R₉, R₁₀, Ra, Rb, X₁ and X₂ have the same meaning as in claim 13.

17. A compound according to claim 16, of of formula VIa 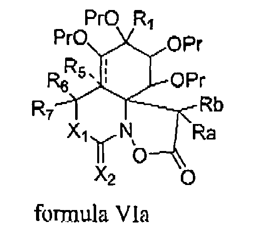 wherein R₁, R₅, R₆, R₇, Ra, Rb, X₁, X₂ and Pr are as defined in claim 16.

18. A compound according to claim 16, of of formula VIa 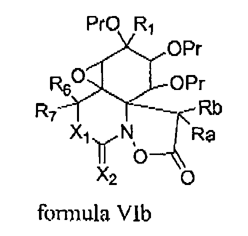 wherein R₁, R₆, R₇, Ra, Rb, X₁, X₂ and Pr are as defined in claim 16.

19. A compound of formula V 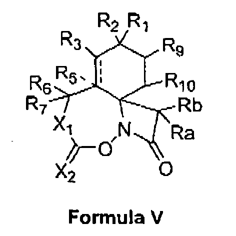 wherein R₁, R₂, R₃, R₅, R₆, R₇, R₉, R₁₀, Ra, Rb, X₁ and X₂ have the same meaning as in claim 13.

20. A compound according to claim 19, which has formula Va 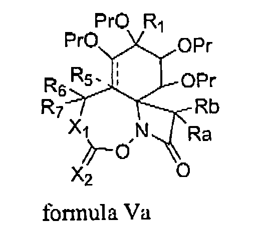 wherein R₁, R₅, R₆, R₇, Ra, Rb, X₁, X₂ and Pr are as defined in claim 19.

21. A compound according to claim 19, which has formula Vb 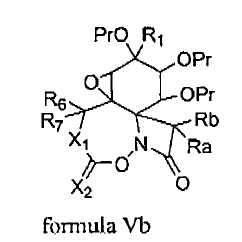 wherein R₁, R₆, R₇, Ra, Rb, X₁, X₂ and Pr are as defined in claim 19.

22. A compound of formula IV 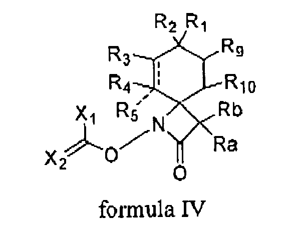 wherein
R₁ and R₂ together are -O or alkenyl; or
R₂ is selected from H, OH or OPr, and R₁ is selected from hydrogen, cyano, substituted or unsubstituted alkyl, alkyl-OPr, substituted or unsubstituted cycloalkyl, substituted or unsubstituted alkenyl, substituted or unsubstituted alkinyl, substituted or unsubstituted aryl, substituted or unsubstituted heterocyclyl;
R₉ and R₁₀ are each independently selected from H, OH, OPr or =O;
R₃ is selected from H, OH, OPr or =O;
R₄ is selected from cyano, substituted or unsubstituted alkyl, OPr, substituted or unsubstituted alkenyl, substituted or unsubstituted alkinyl;
if the bond between C5 and C4a is a single bond, R₅ is selected from H, OH or OPr; or
R₄ and R₅ together are =O; or
R₃ and R₅ or R₃ and R₂ together are -O-, forming an epoxide ring;
Ra and Rb are each independently selected from H, OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino or halogen;
X₁ is selected from NHPrn, NH₂, N(Prn)₂, wherein Prn can be the same or different, OH, OPr, SH or SPrs;
X₂ is selected from NPm, N H, O or S;
Pr is a hydroxyl protecting group
Prn is an amino protecting group
Prs is a sulfur protecting group.

23. A. process for the synthesis of a compound of formula 1: 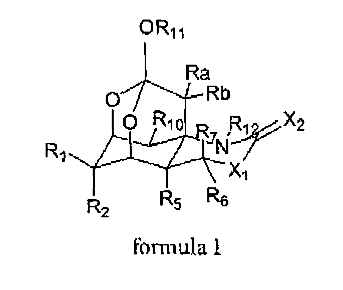 wherein
R₁ and R₂ together are =O or alkenyl; or
R₂ is selected from H, OH or OPr, and R₁ is selected from hydrogen, cyano, substituted or unsubstituted alkyl, alkyl-OPr, substituted or unsubstituted cycloalkyl, substituted or unsubstituted alkenyl, substituted or unsubstituted alkinyl, substituted or unsubstituted aryl, substituted or unsubstituted heterocyclyl;
R₅ is selected from H, OH, OPr;
R₆ and R₇ together are -O or =NPrn; or R₆ and R₇ are each independently selected from H, OH, OPr or cyano;
R₁₀ is selected from H, OH, OPr or -O;
R₁₁ is H, Pr or a negative charge;
R₁₂ is H or Prn;
X₁ is selected from NPrn, NH, O or S;
X₂ is selected from NPrn, NH, ⊕NH₂, O or S;
Ra and Rb are each independently selected from H, OH, OPr or substituted or unsubstituted alkyl, substituted or unsubstituted amino or halogen;
Pr is a hydroxyl protecting group;
Prn is a amino protecting group;
or its salts or tautomers,
comprising the following steps:
a) deprotecting the N-oxide residue of a compound of formula II 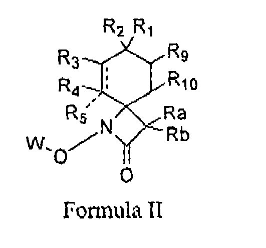 wherein
R₃ and R₉ are each independently selected from H, OH, OPr or=O;
R₄ is selected from cyano, substituted or unsubstituted alkyl, OPr, substituted or unsubstituted alkenyl, substituted or unsubstituted alkinyl;
if the bond between C5 and C4a is a single bond, R₅ is selected from H, OH or OPr; or R₄ and R₅ together are =O;
or R₃ and R₅ or R₃ and R₂ together are -O-, forming and epoxide ring;
W is a group comprising at least a group selected from substituted alkyl, substituted or unsubstituted aryl, substituted or usubstituted heterocyclyl, substituted or unsubstituted alkenyl;
R₁, R₂, R₁₀, Ra, Rb and Pr have the same meaning as in formula 1;
followed by the introduction of a residue of formula III 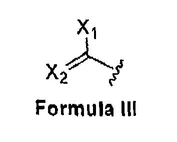 wherein
X₁ is selected from NHPrn, NH₂, N(Prn)₂, wherein Prn can be the same or different, OH, OPr, SH or SPrs;
X₂ is selected from NPrn, NH, O or S;
Prs is a sulfur protecting group;
Pr and Prn have the same meaning as in formula I;
to give a compound of formula IV 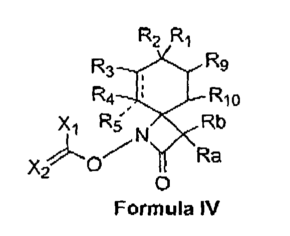 wherein
R₁, R₂, R₃, R₄, R₅, R₉, R₁₀, Ra and Rb have the same meaning as in formula II;
X₁ and X₂ have the same meaning as in formula III;
b) closing the ring between X₁ and either R₄ or C4a of the compound of formula IV to give a compound of formula VI 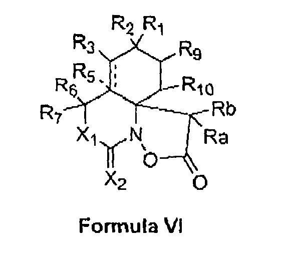 wherein
R₁, R₂, R₃, R₅, R₆, R₇, R₉, R₁₀, X₁, X₂, Ra and Rb have the same meaning as in formula V;
c) opening the 1-aza-2-oxaspiro residue of the compound of formula VI to give a compound of formula VII 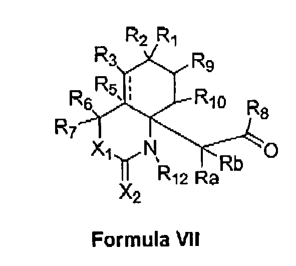 wherein
R₁ and R₂ together are =O or alkenyl; or
R₂ is selected from H, OH or OPr, and R₁ is selected from hydrogen, cyano, substituted or unsubstituted alkyl, alkyl-OPr, substituted or unsubstituted cycloalkyl, substituted or unsubstituted alkenyl, substituted or unsubstituted alkinyl, substituted or unsubstituted aryl, substituted or unsubstituted heterocyclyl;
R₆, and R₇ together are =O or =NPrn; or R₆ and R₇ are each independently selected from H, OH, OPr or cyano;
R₈ is H, OH, ester or amide;
R₉ and R₁₀ are each independently selected from H, OH, OPr or =O;
R₃ is selected from H, OH, OPr or =O,
if the bond between C5 and C4a is a single bond, R₅ is selected from H, OH or OPr; or R₃ and R₅ together are -O-, forming an epoxide ring;
R₁₂ is H or Prn;
Ra and Rb are each independently selected from H, OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino or halogen;
Pr is a hydroxyl protecting group;
Prn is a amino protecting group;
X₁ is selected from NPrn, NH, O or S;
X₂ is selected from NPm, NH, ⊕NH₂, O or S; and
d) formation of the orthoester to give a compound of formula 1 from a compound of formula VII.
